# EUROPEAN PATENT APPLICATION

(11) **EP 4 792 925 A1**
(43) Date of publication of application: **19.08.2026**
(21) Application number: 24877215.4
(22) Date of filing: 09.10.2024
(51) Int. Cl.: B01J 19/00, B01F 33/30, C08F 2/01, C08F 220/14, C08F 220/18, G01N 37/00

(54) **MICROFLUIDIC SYSTEM, PROCESSING METHOD USING MICROFLUIDIC SYSTEM, AND METHOD FOR PRODUCING POLYMER**

(30) Priority: 10.10.2023 JP 2023175299
(71) Applicant: Daicel Corporation, Osaka-shi, Osaka 530-0011 (JP); IMT Taiwan Co., Ltd., Hsinchu City, 30013 (TW)
(72) Inventor: KITAMORI, Takehiko, Hsinchu City, 30013 (TW); CHEN, Chih-Chen, Hsinchu City, 30013 (TW)
(74) Representative: Hoffmann Eitle
(86) International application number: PCT/JP2024/036190
(87) International publication number: WO 2025/079620

(57) **Abstract**

A microfluidic system includes a plurality of chip-type microfluidic devices in microchannel is formed, the microfluidic system including: one or more mixing devices, each of which is a microfluidic device that has a mixing channel configured to mix a plurality of different fluids and an introduction unit configured to introduce at least one of the plurality of different fluids into the mixing channel; and one or more other-process devices, each of which is a microfluidic device configured to perform, within a microchannel, a chemical, biological, or physical process other than a mixing process on a mixed fluid resulting from mixing the plurality of different fluids in the mixing channel.

## Description

### Technical Field

The present disclosure relates to a microfluidic system, a processing method using the microfluidic system, and a method for producing a polymer.

### Background Art

In recent years, in the fields of chemical synthesis and chemical analysis, the use of microfluidic devices has been promoted to perform unit operations such as chemical, biological, or physical processes on fluids. A microfluidic device is a device including a minute flow path (microchannel) that has a channel width on the order of micrometers and performs a unit operation on a fluid flowing through the microchannel. In particular, a microfluidic device intended for performing a chemical reaction in the microchannel as the unit operation is also referred to as a microreactor.

A microreactor mixes a plurality of different types of fluids (different fluids) introduced into a minute flow path, and brings the fluids into contact with each other, thereby causing a chemical reaction. In the microreactor, since the minute flow path serves as a reaction field, the plurality of different types of fluids each form a thin laminar flow, and mixing and reaction of the respective fluids are rapidly performed by molecular diffusion at the contact interface. In addition, the microreactor has an advantage that the efficiency of heat transfer and heat conduction is higher than that of a conventional batch-type reaction apparatus using a large tank because the effect of surface area relative to the volume of the fluids becomes significant.

As a technology using a microreactor, Patent document 1 discloses a technology for controlling the uniformity (particularly, the molecular weight distribution) of a resulting polymer by using a microreactor including a first introduction port for introducing a monomer component and another introduction port located downstream of the first introduction port, the microreactor being capable of introducing the monomer component into both the first introduction port and the other introduction port.

### Citation List

### Patent document

Patent document 1: Japanese Patent Laid-Open No. 2020-29518

### Summary of the Invention

### Problems to be solved by the invention

When a chemical, biological, or physical process such as a chemical reaction is allowed to proceed using a plurality of components by a microfluidic system including a microfluidic device, the plurality of components to be subjected to the chemical, biological, or physical process are usually supplied into the chemical, biological, or physical process system by mixing two or more types of fluids containing some of the components. In a state where these fluids are not uniformly mixed, that is, in a state where there is a bias in the concentrations of the various components, the intended result may not be obtained. When a chemical reaction is performed as a chemical process, there is a concern that problems may arise such that the chemical reaction does not proceed uniformly, and as a result, the quality of the reaction product cannot be ensured (for example, a reaction product having characteristics such as a desired chemical structure and molecular weight cannot be obtained). Therefore, to obtain the intended result in a microfluidic device, it is required to allow the chemical, biological, or physical process to proceed after uniformly mixing the various components.

The technology of the present disclosure has been made in view of the above circumstances, and an object thereof is to provide a microfluidic system including a microfluidic device, the microfluidic system being capable of realizing a state in which various components to be subjected to chemical, biological, or physical processes are uniformly mixed, and further being capable of allowing the chemical, biological, or physical processes to proceed from that state.

### Means for solving the Problems

As a result of intensive studies to attain the object, the present inventors have found that the object can be attained by using a mixing device configured to mix a plurality of different fluids and an other-process device configured to perform a chemical, biological, or physical process other than mixing. That is, the gist of the present disclosure is as follows.
[1] A microfluidic system including a plurality of chip-type microfluidic devices in which a microchannel is formed, the microfluidic system including:
   one or more mixing devices, each of which is a microfluidic device that has a mixing channel configured to mix a plurality of different fluids and an introduction unit configured to introduce at least one of the plurality of different fluids into the mixing channel; and
   one or more other-process devices, each of which is a microfluidic device configured to perform, within a microchannel, a chemical, biological, or physical process other than a mixing process on a mixed fluid resulting from mixing the plurality of different fluids in the mixing channel.
[2] The microfluidic system according to [1], wherein the chemical, biological, or physical process performed in the other-process devices is at least one selected from chemical reaction, extraction, distillation, concentration, solid-phase extraction, particle separation or classification, crystallization, and cell culture.
[3] The microfluidic system according to [1],
   wherein the other-process devices are each a reaction device in which a chemical reaction is performed, the reaction device including a reaction channel configured to allow a chemical reaction of a reactant contained in the mixed fluid resulting from mixing the plurality of different fluids in the mixing channel to proceed; and
   wherein the one or more mixing devices and the one or more reaction devices are arranged in series such that the mixing channel and the reaction channel communicate with each other.
[4] The microfluidic system according to [3], including a plurality of the mixing devices and a plurality of the reaction devices,
   wherein the plurality of mixing devices and the plurality of reaction devices are arranged alternately in series.
[5] The microfluidic system according to [3] or [4],
   wherein the plurality of different fluids include at least a fluid containing a polymerization initiator and a fluid containing one or more monomers;
   wherein the fluid containing the one or more monomers is introduced into the mixing channel of the mixing device from the introduction unit; and
   wherein, in the reaction channel of the reaction device, the one or more monomers are polymerized in the presence of the polymerization initiator.
[6] The microfluidic system according to any one of [3] to [5], further including a reaction-promoting unit configured to promote a chemical reaction of a reactant contained in the mixed fluid in the reaction channel.
[7] The microfluidic system according to [6], wherein the reaction-promoting unit includes at least one selected from a heating unit, a light-irradiation unit, a vibration-energy-imparting unit, and a voltage-application unit.
[8] The microfluidic system according to any one of [3] to [7], wherein a channel width of the mixing channel and a channel width of the reaction channel are each 1000 µm or less.
[9] The microfluidic system according to any one of [1] to [8],
   wherein the one or more mixing devices and the one or more other-process devices are arranged in series; and
   wherein, in at least one pair of a mixing device and an other-process device that are adjacent to each other in a flow direction, the mixing device and the other-process device are arranged side by side in a direction perpendicular to a plane in which the microfluidic device extends.
[10] The microfluidic system according to [9], further including a partition member having heat-insulating properties, the partition member being disposed between the mixing device and the other-process device in the at least one pair of the mixing device and the other-process device.
[11] A processing method including using a microfluidic system including a plurality of chip-type microfluidic devices in which a microchannel is formed,
   the microfluidic system including:
   one or more mixing devices, each of which is a microfluidic device that has a mixing channel configured to mix a plurality of different fluids and an introduction unit configured to introduce at least one of the plurality of different fluids into the mixing channel; and
   one or more other-process devices, each of which is a microfluidic device configured to perform, within a microchannel, a chemical, biological, or physical process other than a mixing process on a mixed fluid resulting from mixing the plurality of different fluids in the mixing channel.
[12] The processing method according to [11], wherein the chemical, biological, or physical process performed in the other-process devices is at least one selected from chemical reaction, extraction, distillation, concentration, solid-phase extraction, particle separation or classification, crystallization, and cell culture.
[13] The processing method according to [11],
   wherein the other-process devices are each a reaction device in which a chemical reaction is performed, the reaction device including a reaction channel configured to allow a chemical reaction of a reactant contained in the mixed fluid resulting from mixing the plurality of different fluids in the mixing channel to proceed,
   wherein the one or more mixing devices and the one or more reaction devices are arranged in series such that the mixing channel and the reaction channel communicate with each other.
[14] The processing method according to [13],
   wherein the microfluidic system includes a plurality of the mixing devices and a plurality of the reaction devices; and
   wherein the plurality of mixing devices and the plurality of reaction devices are arranged alternately in series.
[15] The processing method according to [13] or [14],
   wherein the plurality of different fluids include at least a fluid containing a polymerization initiator and a fluid containing one or more monomers;
   wherein the fluid containing the one or more monomers is introduced into the mixing channel of the mixing device from the introduction unit; and
   wherein, in the reaction channel of the reaction device, the one or more monomers are polymerized in the presence of the polymerization initiator.
[16] The processing method according to any one of [13] to [15], wherein the microfluidic system further includes a reaction-promoting unit configured to promote a chemical reaction of a reactant contained in the mixed fluid in the reaction channel.
[17] The processing method according to [16], wherein the reaction-promoting unit includes at least one selected from a heating unit, a light-irradiation unit, a vibration-energy-imparting unit, and a voltage-application unit.
[18] The processing method according to any one of [11] to [17],
   wherein the one or more mixing devices and the one or more other-process devices are arranged in series; and
   wherein, in at least one pair of a mixing device and an other-process device that are adjacent to each other in a flow direction, the mixing device and the other-process device are arranged side by side in a direction perpendicular to a plane in which the microfluidic device extends.
[19] The processing method according to [18], wherein a partition member having heat-insulating properties is disposed between the mixing device and the other-process device in the at least one pair of the mixing device and the other-process device.
[20] A method for producing a polymer, the method including using a microfluidic system including a plurality of chip-type microfluidic devices in which a microchannel is formed,
   the microfluidic system including:
   a plurality of mixing devices, each of which is a microfluidic device that has a mixing channel configured to mix a plurality of different fluids including at least a fluid containing a polymerization initiator and a fluid containing one or more monomers, and an introduction unit configured to introduce at least the one or more monomers among the plurality of different fluids into the mixing channel; and
   one or more reaction devices, each of which is a microfluidic device that has a reaction channel configured to allow a polymerization reaction of the one or more monomers to proceed in the presence of the polymerization initiator by the plurality of different fluids mixed in the mixing channel,
   wherein the plurality of mixing devices and the one or more reaction devices are arranged in series such that the mixing channel and the reaction channel communicate with each other.

### Effects of the invention

According to the technology of the present disclosure, it becomes possible to provide a microfluidic system including a microfluidic device, the microfluidic system being capable of realizing a state in which various components to be subjected to chemical, biological, or physical processes are uniformly mixed, and further being capable of allowing the chemical, biological, or physical processes to proceed from that state.

### Brief Description of the Drawings

[Fig. 1] Fig. 1 is a configuration diagram of a polymer production system according to the present embodiment.
[Fig. 2] Fig. 2 is a plan diagram of a mixing device according to the present embodiment.
[Fig. 3] Fig. 3 is a plan diagram of a reaction device according to the present embodiment.
[Fig. 4] Fig. 4 is a configuration diagram of a polymer production system according to a comparative example.
[Fig. 5] Fig. 5 is a configuration diagram of a polymer production system according to a modification example of the present embodiment.

### Mode for Carrying Out the Invention

Each configuration and combinations thereof in each embodiment are merely examples, and additions, omissions, substitutions, and other modifications of the configurations can be made as appropriate without departing from the spirit of the present disclosure. The present disclosure is not limited by the embodiments and is limited only by the scope of the claims. In addition, each aspect disclosed in the present specification can be combined with any other feature disclosed in the present specification. Furthermore, a numerical range expressed using "to" means a range including the numerical values described before and after "to" as the lower limit and the upper limit, respectively, and "A to B" means A or more and B or less.

One embodiment of the present disclosure is a microfluidic system including a plurality of chip-type microfluidic devices in which a microchannel is formed, the microfluidic system including:
one or more mixing devices, each of which is a microfluidic device that has a mixing channel configured to mix a plurality of different fluids and an introduction unit configured to introduce at least one of the plurality of different fluids into the mixing channel; and
one or more other-process devices, each of which is a microfluidic device configured to perform, within a microchannel, a chemical, biological, or physical process other than a mixing process on a mixed fluid resulting from mixing the plurality of different fluids in the mixing channel.

### <Microfluidic device>

A microfluidic device is a device in which a minute flow channel (microchannel) having a channel width on the order of micrometers is formed. The microfluidic device according to the present disclosure is not particularly limited and may be a chip-type microfluidic device in which a minute flow channel is formed inside or on the surface of a substrate, or may be a tube-type microfluidic device in which a minute flow channel is formed inside a tube. In addition, the microfluidic device can be referred to as a micromixer when used for mixing, and can be referred to as a microreactor when used for chemical reactions, depending on the purpose.

The microchannel may be formed inside the substrate or may be formed on the surface of the substrate. The cross-sectional shape of the microchannel is not particularly limited and may be appropriately selected depending on the purpose; examples thereof include circular, rectangular, semicircular, and triangular shapes. In addition, the width (inner diameter) or cross-sectional area of the microchannel is not particularly limited as long as the effects of the technology according to the present disclosure are not impaired, and may be appropriately selected depending on the purpose. For example, the channel width is preferably 15 mm or less, more preferably 20 µm or more and 1000 µm or less, and still more preferably 100 µm or more and 500 µm or less. When the channel width exceeds 15 mm, the surface area per unit volume becomes small, and as a result, rapid mixing and removal of reaction heat may become difficult. When the channel width is more than 1000 µm, the diffusion distance of molecules becomes larger, thereby reducing mixing efficiency and potentially degrading the function of the microfluidic device. In addition, when the channel width is less than 20 µm, the pressure loss of the liquid flowing through the channel increases, and a high-pressure-resistant pump is required for liquid feeding, which may increase production costs. The cross-sectional area of the channel is preferably 0.0001 mm² or more and 225 mm² or less, more preferably 0.0003 mm² or more and 1 mm² or less, and still more preferably 0.01 mm² or more and 0.25 mm² or less. The length (channel length) of the channel formed in the microfluidic device is not particularly limited and may be appropriately set depending on the mixing time or reaction time. For example, in polymer production, the channel length of the mixing channel in the mixing device is preferably 10 mm or more and 3000 mm or less, more preferably 20 mm or more and 2000 mm or less, and still more preferably 50 mm or more and 1000 mm or less. In polymer production, the channel length of the reaction channel in the reaction device is preferably 10 mm or more and 500 m or less, more preferably 20 mm or more and 10000 mm or less, and still more preferably 50 mm or more and 5000 mm or less. The flow rate of the liquid flowing through the microchannel of the microfluidic device is not particularly limited and may be appropriately selected depending on the purpose. For example, in polymer production, the flow rate is preferably 0.000001 mL/min or more and 10 mL/min or less, more preferably 0.00001 mL/min or more and 0.1 mL/min or less, and still more preferably 0.0001 mL/min or more and 0.05 mL/min or less. When the flow rate is within the above range, rapid mixing of the monomer component and the polymerization initiator tends to be achieved, and pressure loss also tends to be suppressed. Note that the above flow rate refers to the flow rate of the fluid immediately after being introduced into the microchannel.

### <Mixing device>

A mixing device is a microfluidic device in which at least a mixing process is performed as a unit operation. The plurality of different fluids subjected to the mixing process in the mixing device is not particularly limited, and examples thereof include liquid-phase mixing, gas-liquid mixing, and slurry mixing. The mixing device has, as a microchannel, a mixing channel configured to mix the plurality of different fluids. As the plurality of different fluids flow through the mixing channel, the mixing process of the plurality of different fluids proceeds.

### <Other-process device>

An other-process device is a microfluidic device in which a chemical, biological, or physical process other than a mixing process is performed. In the present specification, the term "chemical, biological, or physical process" refers to "one or more processes selected from the group consisting of a chemical process, a biological process, and a physical process". The chemical, biological, or physical process performed in the other-process device is not particularly limited, and examples thereof include liquid-phase reaction, gas-liquid reaction, extraction, distillation, concentration, slurry reaction, solid-phase extraction, particle separation or classification, crystallization, and cell culture. The other-process device performs, in a microchannel, a unit operation other than a mixing process on a mixed fluid obtained by mixing a plurality of different fluids in the mixing channel of the mixing device. The process performed in the other-process device may be any one or more chemical, biological, or physical processes other than a mixing process, and therefore may further include a mixing process in addition to the one or more chemical, biological, or physical processes.

### <Reaction device>

A reaction device is an other-process device in which, as a chemical, biological, or physical process other than a mixing process, at least a chemical reaction is performed. The chemical reaction performed in the reaction device is not particularly limited, and examples thereof include solid-phase reaction, slurry mixing or reaction, liquid-phase reaction, liquid-phase mixing, and gas-liquid reaction.

### <Reaction-promoting unit>

The reaction-promoting unit is not particularly limited and may be appropriately selected depending on the desired chemical reaction. Examples of the reaction-promoting unit include a heating unit, a light-irradiation unit, a vibration-energy-imparting unit, and a voltage-application unit. Examples of the heating unit include a heater and a microwave irradiation device. Examples of the light-irradiation unit include an LED, an organic light-emitting device (OLED), a laser, and an arc lamp. Examples of the vibration-energy-imparting unit include an ultrasonic generator and a piezoelectric transducer. Examples of the voltage-application unit include electrodes. The reaction-promoting unit may include at least one of the various units described above, and may also be configured as a combination of a plurality of these units. For example, the reaction-promoting unit may heat the fluid in the reaction channel using a microwave irradiation device serving as a heating unit or may irradiate the fluid in the reaction channel with light using an illumination device serving as a light-irradiation unit that employs various light sources.

### <Different fluid>

In the microfluidic system according to the present embodiment, a chemical reaction of a reactant contained in a mixed fluid (i.e., a reaction system) of a plurality of different fluids proceeds in the reaction channel of the reaction device. In the present embodiment, the plurality of different fluids is allowed to flow through the mixing channel of the mixing device, thereby yielding a mixed fluid in which the plurality of different fluids are uniformly mixed. As a result, the reaction can proceed uniformly within the same reaction system, suitably even across a plurality of lots. The plurality of different fluids may be liquids or gases, preferably liquids.

In the present specification, the state in which a plurality of different fluids is uniformly mixed refers, in the case where the mixed fluid composed of a single phase, to a state in which the concentrations of the respective components in the mixed fluid are highly uniform and exhibit no or only minimal concentration variation. In the case where the mixed fluid composed of two or more phases including a liquid phase, the state refers to a state in which the concentrations of the respective components in the liquid phase are highly uniform and in which the other phase(s) are uniformly dispersed in the liquid phase. Example of the mixed fluid composed of two or more phase including a liquid phase preferably include an emulsion (such as an oil-in-water emulsion or a water-in-oil emulsion), which is a dispersion system where a liquid dispersoid is dispersed in a liquid dispersion medium; and a suspension, which is a dispersion system where a solid dispersoid is dispersed in a liquid dispersion medium. The term "uniform" and "highly uniform" as used herein refer to a state of being more "uniform" and "highly uniform," respectively, in comparison with the case of a conventional microfluidic system that lacks a mixing device configured to mix a plurality of different fluids.

Each of the plurality of different fluids is a fluid for a supply of a component necessary for the progress of a chemical reaction to a reaction system. The component necessary for the progress of the chemical reaction includes one or more reactants and may further include another component necessary for the progress of the chemical reaction (hereinafter sometimes simply referred to as the "other component"), and preferably include such other component. Each of the plurality of different fluids contains one or more other components necessary for the progress of the chemical reaction. In other words, among the plurality of different fluids, one fluid contains one or more reactants, and one or more other fluids each contain one or more reactants and/or one or more other components. Among the plurality of different fluids to be mixed in a mixing device disposed immediately downstream of a reaction device, one or more fluids contain a reaction product obtained through a chemical reaction in the reaction device. The reaction product may be a reactant to be subjected to a chemical reaction in a reaction device disposed further downstream of the mixing device, or may be a component that lacks reactivity and causes no chemical reaction (i.e., a component other than the component necessary for the progress of the chemical reaction).

When any of the plurality of different fluids contains another component necessary for the progress of the chemical reaction, the other component necessary for the progress of the chemical reaction may be contained in the one fluid, or may be contained in both the one fluid and the one or more other fluids. However, the other component is preferably contained in the one or more other fluids.

The other component necessary for the progress of the chemical reaction may be appropriately selected, if needed, from components known or conventionally used in the intended chemical reaction. Examples of the other component necessary for the progress of the chemical reaction include an initiator, a catalyst, a catalyst auxiliary, a solvent, a reaction reagent such as an acid, a base, or water, and an additive such as a surfactant.

Which component each of the plurality of different fluids supplies may be determined based on the convenience of mixing (for example, various conditions such as properties of the reactant, properties of the other component, and the number of components necessary for the progress of the chemical reaction). The number of components contained in one fluid is not particularly limited and is usually one or more and five or less, preferably one or more and four or less, and more preferably two or more and three or less. The number of the plurality of different fluids is not particularly limited as long as it is 2 or more, but is preferably 2 or more and 5 or less, more preferably 2 or more and 3 or less, and still more preferably 2.

To describe more specifically, in the case of a reaction between a compound A and a compound B in a solvent A in the presence of the catalyst A, the compound A and the compound B each correspond to the reactant, and the solvent A and the catalyst A each correspond to the other component necessary for the progress of the chemical reaction. As long as each of the plurality of different fluids contain one or more of these components, the type and number of components contained therein are not particularly limited. For example, the plurality of different fluids may be three fluids: a solution of the compound A dissolved in the solvent A; a solution of the compound B dissolved in the solvent A; and a solution of the catalyst A dissolved in the solvent A. Alternatively, for example, the plurality of fluids may be two fluids: a fluid composed of the compound A and the compound B; and a solution of the catalyst A dissolved in the solvent A.

When the chemical reaction proceeds in the absence of the other component necessary for the progress of the chemical reaction, none of the mixed fluid of a plurality of different fluids contains the other component necessary for the progress of the chemical reaction. Such a case is, for example, a case where a chemical reaction between a reactant A and a reactant B proceeds in a reaction system consisting of the reactant A and the reactant B. In this case, in a mixing device, one of the reactant A, which serves as one fluid, and the reactant B, which serves as the other fluid, may be allowed to flow through a predetermined processing channel, and the other may be introduced from an introduction unit.

Examples of the chemical reaction include a synthesis reaction such as a synthesis reaction of an organic compound, a synthesis reaction of an inorganic compound, or a synthesis reaction of an organic-inorganic hybrid material; and a decomposition reaction such as a decomposition reaction of an organic compound, a decomposition reaction of an inorganic compound, or a decomposition reaction of an organic-inorganic hybrid material. Among them, the chemical reaction is preferably a synthesis reaction, more preferably a synthesis reaction of an organic compound, and still more preferably a synthesis reaction of a polymer. In these chemical reactions, the advantages of the microfluidic system according to the present embodiment, in which the chemical reaction proceeds sufficiently to ensure the quality of the reaction product, can be maximized. However, since a sufficient progress of the reaction can be achieved by ensuring an appropriate residence time in the reaction channel regardless of the type of chemical reaction, the technical effects of the present disclosure can also be obtained in a chemical reaction other than those described above. Therefore, the chemical reaction is not limited to the chemical reaction described above.

The microfluidic system according to the present embodiment allows the chemical reaction to proceed uniformly, and therefore, in the production of a polymer, a polymer having a small polydispersity index Mw/Mn (Mw: weight-average molecular weight, Mn: number-average molecular weight) can be produced. Accordingly, the microfluidic system according to the present embodiment is suitable for producing a polymer, and is more suitable for producing a polymer for a photoresist. Hereinafter, an example of the plurality of different fluids in the case where a polymer is produced through a radical polymerization reaction of a monomer is described.

In this example, a polymer is produced according to the following procedures (i) to (v) using a microfluidic device in which four microfluidic devices-a mixing device, a reaction device, a mixing device, and a reaction device-are arranged in series in this order from the upstream side.
(i) In the mixing device, which is the microfluidic device arranged at the most upstream position, a monomer solution prepared by dissolving a monomer in a solvent and an initiator solution prepared by dissolving a radical polymerization initiator in a solvent are mixed to prepare a mixed fluid. Here, the monomer corresponds to a reactant, and the solvent and the radical polymerization initiator correspond to other components. Furthermore, the monomer solution and the initiator solution correspond to a plurality of different fluids.
(ii) Next, the mixed fluid obtained in (i) above is introduced into the reaction device, which is the microfluidic device arranged second from the upstream side, to perform a radical polymerization reaction of the monomer. In this reaction device, most of the monomer is consumed, and the reaction mixture obtained after the polymerization reaction contains a small amount of monomer, a radical polymerization initiator (and/or a radical generated from the radical polymerization initiator; in the present specification, the term "radical polymerization initiator" is to be understood as also including a radical generated from the radical polymerization initiator), a solvent, and a polymer as the reaction product.
(iii) The reaction mixture obtained in (ii) above is introduced into a mixing channel of the mixing device, which is the microfluidic device arranged third from the upstream side, a monomer solution prepared by dissolving a monomer in a solvent is further introduced from an introduction port, and these fluids are mixed in the mixing device to prepare a mixed fluid. Here, the monomer corresponds to the reactant, and the solvent and the radical polymerization initiator correspond to other components. The monomer solution and the reaction mixture correspond to a plurality of different fluids.
(iv) Subsequently, the mixed fluid obtained in (iii) above is introduced into a reaction device, which is a microfluidic device arranged fourth from the upstream side, to perform a radical polymerization of the monomer.
(v) A polymer is recovered from the reaction mixture discharged from the reaction device in (iv) above.

As in the above example, in the case where a monomer solution is added at each stage in a multistage polymer synthesis, the monomer mixed in one mixing device and the monomer to be mixed in one or more other mixing devices (in the above example, the monomer introduced into the mixing device in (i) and the monomer introduced into the mixing device in (iii)) may be the same or different, but are preferably the same from the viewpoint of mass production of the same polymer.

In the above example, the microfluidic device used is configured by alternately arranging two mixing devices and two reaction devices. In a modification example in which three or more mixing devices and three or more reaction devices are alternately arranged, a polymer can be produced by repeating the same operations as in (iii) and (iv). In another modification example in which the monomer solution introduced into the mixing device in (iii) is replaced with a fluid containing a chemical modifying agent, a polymer in which one or more of the terminal, main chain, and side chain of the polymer obtained in (ii) are modified can be obtained.

In the production of a polymer, a solution prepared by dissolving a chain transfer agent in a solvent may additionally be introduced into the mixing device to prepare a mixed fluid containing the chain transfer agent, thereby enabling production of a polymer having a smaller polydispersity index Mw/Mn.

The quality of the polymer can be evaluated, for example, based on the polydispersity index Mw/Mn. In general, the narrower the molecular-weight distribution of the polymer-that is, the smaller the polydispersity index Mw/Mn-the smaller the variation in the molecular weight of the polymer, thereby making it easier to obtain a polymer having desired properties. In the production of a polymer, the microfluidic system according to the present embodiment is suitable for producing a polymer having a polydispersity index Mw/Mn of 1.3 or more and 3.0 or less, more suitable for producing a polymer having a polydispersity index Mw/Mn of 1.3 or more and 2.0 or less, still more suitable for producing a polymer having a polydispersity index Mw/Mn of 1.3 or more and 1.9 or less, even more suitable for producing a polymer having a polydispersity index Mw/Mn of 1.3 or more and 1.8 or less, particularly suitable for producing a polymer having a polydispersity index Mw/Mn of 1.3 or more and 1.7 or less, and especially suitable for producing a polymer having a polydispersity index Mw/Mn of 1.3 or more and 1.6 or less.

The quality of the polymer can also be evaluated based on the weight-average molecular weight Mw and/or the number-average molecular weight. In the production of a polymer, the microfluidic system according to the present embodiment is suitable for producing a polymer having a weight-average molecular weight Mw of 500 or more and 8000 or less, more suitable for producing a polymer having a weight-average molecular weight Mw of 500 or more and 7000 or less, and still more suitable for producing a polymer having a weight-average molecular weight Mw of 1000 or more and 6000 or less. In the production of a polymer, the microfluidic system according to the present embodiment is suitable for producing a polymer having a number-average molecular weight Mn of 500 or more and 4500 or less, more suitable for producing a polymer having a number-average molecular weight Mn of 1000 or more and 4000 or less, and still more suitable for producing a polymer having a number-average molecular weight Mn of 2000 or more and 3500 or less.

The weight-average molecular weight Mw and the number-average molecular weight Mn are measured by gel permeation chromatography (GPC).

The polydispersity index Mw/Mn is calculated by measuring the weight-average molecular weight Mw and the number-average molecular weight Mn of the polymers by gel permeation chromatography (GPC) and dividing the former by the latter. As the measurement conditions for the GPC, the following conditions are adopted.
Apparatus: GPC system (manufactured by Shimadzu Corporation)
System controller: SIL-20A (manufactured by Shimadzu Corporation)
Pump: LC-20AD (manufactured by Shimadzu Corporation)
Degasser: DGU-20A3R (manufactured by Shimadzu Corporation)
Column oven: CTO-20AC (manufactured by Shimadzu Corporation)
RI detector: RID-20A (manufactured by Shimadzu Corporation)
Column: GPC KF-806L (column size: 8.0 mm (ID) × 300 mm (L), manufactured by Resonac Corporation) × 3 columns
Guard column: KF-G (column size: 4.6 mm (ID) × 10 mm (L), manufactured by Resonac Corporation)
Column temperature: 40°C
Cell temperature: 40°C
Eluent: tetrahydrofuran
Eluent flow rate: 0.8 mL/min
Injection volume: 35 µL
Analysis time: 60 min
Sample: 5 wt% tetrahydrofuran solution
Calibration standard: polystyrene calibration kit S-M-10 (manufactured by Agilent Technologies, Inc.)

The monomer, the radical polymerization initiator, the solvent, and the chain transfer agent used in the radical polymerization reaction of the monomer as described above are described in more detail below.

### (Monomer)

The type of monomer, the number of monomer types, and the monomer mixing ratio in a case where two or more monomer types are used, the monomers are selected depending on the polymer to be produced. Accordingly, the monomer may be used as a single type of monomer, or two or more types may be used in any combination and ratio. When two or more monomers are used to perform a copolymerization reaction, examples of the type of copolymerization sequence include, but are not particularly limited to, random copolymerization, alternating copolymerization, block copolymerization, and graft copolymerization. The structure of the resulting polymer is not particularly limited, and the polymer may be linear, branched, or may have a cyclic structure.

The monomer is not particularly limited as long as it is a monomer capable of undergoing polymerization (hereinafter also referred to as "polymerizable monomer"), and examples thereof include polymerizable monomers disclosed in, for example, Japanese Patent Laid-Open No. 2018-149791 and Japanese Patent Laid-Open No. 2010-194983. Specific examples of the polymerizable monomer include at least one monomer selected from the group consisting of a polymerizable monomer containing a (meth)acryloyl group, a polymerizable monomer containing an isocyanate group, a polymerizable monomer containing a carboxyl group, a polymerizable monomer containing a hydroxyl group, a polymerizable monomer containing an epoxy group, a polymerizable monomer containing an oxazoline group, a polymerizable monomer containing a maleimide group, a polymerizable monomer containing an amino group, a styrenic monomer, a fluorine-containing vinyl monomer, a silicon-containing vinyl-based monomer, a conjugated diene-based monomer, an aromatic vinyl monomer, a carboxylic acid vinyl ester, an olefinic monomer, a vinyl halide, a vinylidene halide, an allyl halide, vinyl ethyl ether, and the like.

These monomers may be used singly or in combination of two or more. Further, monomers listed below may be derivatives thereof.

The monomers may be synthesized monomers or commercially available monomers.

In the present specification, the terms "(meth)acryloyl", "meth(acryl)", and "(meth)acrylate" mean "acryloyl and/or methacryloyl", "methacryl and/or acryl", and "acrylate and/or methacrylate", respectively.

Examples of the polymerizable monomer containing a (meth)acryloyl group include methyl (meth)acrylate, ethyl (meth)acrylate, n-propyl (meth)acrylate, isopropyl (meth)acrylate, n-butyl (meth)acrylate, isobutyl (meth)acrylate, tert-butyl (meth)acrylate, n-pentyl (meth)acrylate, n-hexyl (meth)acrylate, cyclohexyl (meth)acrylate, n-heptyl (meth)acrylate, n-octyl (meth)acrylate, 2-ethylhexyl (meth)acrylate, nonyl (meth)acrylate, decyl (meth)acrylate, dodecyl (meth)acrylate, phenyl (meth)acrylate, tolyl (meth)acrylate, benzyl (meth)acrylate, 2-methoxyethyl (meth)acrylate, 3-methoxybutyl (meth)acrylate, stearyl (meth)acrylate, γ-(methacryloyloxypropyl)trimethoxysilane, trifluoromethylmethyl (meth)acrylate, 2-trifluoromethylethyl (meth)acrylate, 2-perfluoroethylethyl (meth)acrylate, 2-perfluoroethyl-2-perfluorobutylethyl (meth)acrylate, perfluoroethyl (meth)acrylate, perfluoromethyl (meth)acrylate, diperfluoromethylmethyl (meth)acrylate, 2-perfluoromethyl-2-perfluoroethylethyl (meth)acrylate, 2-perfluorohexylethyl (meth)acrylate, 2-perfluorodecylethyl (meth)acrylate, and 2-perfluorohexadecylethyl (meth)acrylate. In addition to these monomers, a monomer having a chemical structure that imparts photosensitivity, as described later, may also be included.

Examples of the aromatic vinyl monomer include an alkylstyrene such as styrene; o-methylstyrene, m-methylstyrene, p-methylstyrene, 2,4-dimethylstyrene, p-ethylstyrene, p-isopropylstyrene, p-n-butylstyrene, p-tert-butylstyrene, α-methylstyrene, and α-methyl-p-methylstyrene; an alkoxystyrene such as o-methoxystyrene, m-methoxystyrene, p-methoxystyrene, or p-tert-butoxystyrene; a halostyrene such as o-chlorostyrene, m-chlorostyrene, p-chlorostyrene, or p-bromostyrene; a hydroxystyrene such as o-hydroxystyrene, m-hydroxystyrene, p-hydroxystyrene, or 3,5-dihydroxystyrene; and styrenesulfonic acid or an alkali metal salt thereof.

Examples of the carboxylic acid vinyl ester include a carboxylic acid vinyl ester having 3 or more 10 or less carbon atoms, such as vinyl formate, vinyl acetate, vinyl propionate, or vinyl pivalate.

Examples of the conjugated diene-based monomer include a conjugated diene having 4 or more and 16 or less carbon atoms, such as butadiene, isoprene, chloroprene, 1,3-pentadiene, 2,3-dimethyl-1,3-butadiene, piperine, 3-butyl-1,3-octadiene, or 1-phenyl-1,3-butadiene.

Examples of the olefinic monomer include an alkene having 2 or more and 10 or less carbon atoms, such as ethylene, propylene, 1-butene, 2-butene, or isobutene.

Examples of the vinyl halide include vinyl fluoride, vinyl chloride, and vinyl bromide.

Examples of the vinylidene halide include vinylidene fluoride, vinylidene chloride, and vinylidene bromide.

When the polymer to be produced is a polymer for a photoresist, a monomer having a chemical structure that imparts photosensitivity may be used in place of, or together with, the monomers described above. For example, in the case of producing a polymer for a chemically amplified photoresist using a photoacid generator, the monomer preferably includes a monomer having a group (sometimes referred to as an "acid-decomposable group") in which a portion of the group is cleaved by the action of an acid to form a polar group. As a result, the polymer (photoresist resin) exhibits increased polarity due to the action of the acid and in turn has increased solubility in an alkaline developer, thereby enabling pattern formation.

Examples of the polar group include an acidic group and an alcoholic hydroxyl group. Examples of the acidic group include a phenolic hydroxyl group; a carboxyl group; a fluorinated alcohol group such as a hexafluoro-2-hydroxyisopropyl group; a sulfonic acid group; a sulfonamide group; a sulfonylimide group; an (alkylsulfonyl) (alkylcarbonyl)methylene group; an (alkylsulfonyl) (alkylcarbonyl)imide group; a bis(alkylcarbonyl)methylene group; a bis(alkylcarbonyl)imide group; a bis(alkylsulfonyl)methylene group; a bis(alkylsulfonyl)imide group; a tris(alkylcarbonyl)methylene group; and a tris(alkylsulfonyl)methylene group. Among them, the polar group is preferably a carboxyl group, a fluorinated alcohol group, or a sulfonic acid group.

Examples of the acid-decomposable group preferably include a group in which a hydrogen atom of the polar group is substituted with a group that is cleaved by the action of an acid. Examples of the acid-decomposable group include a group represented by -C(R^{I})(R^{II})(R^{III}) and a group represented by -C(R^{IV})(R^{V})(OR^{VI}). In these formulae, R^{I} to R^{III} and R^{VI} each independently represent an alkyl group, a cycloalkyl group, an aryl group, an aralkyl group, or an alkenyl group. R^{IV} and R^{V} each independently represent a hydrogen atom, an alkyl group, a cycloalkyl group, an aryl group, an aralkyl group, or an alkenyl group. At least two groups of R^{I} to R^{III} may be bonded to each other to form a ring. R^{IV} and RV may also be bonded to each other to form a ring.

The lower limit of the number of carbon atoms in the acid-decomposable group is not particularly limited, but is preferably 4 or more, and more preferably 5 or more. The upper limit of the number of carbon atoms in the acid-decomposable group is not particularly limited, but is preferably 20 or less.

The alkyl group represented by each of R^{I} to R^{VI} is preferably an alkyl group having 1 or more and 8 or less carbon atoms. Examples of the alkyl group having 1 or more and 8 or less carbon atoms include a methyl group, an ethyl group, a propyl group, an n-butyl group, a sec-butyl group, a tert-butyl group, a hexyl group, and an octyl group.

The cycloalkyl group represented by each of R^{I} to R^{VI} may be a monocyclic hydrocarbon group or a polycyclic (bridged cyclic) hydrocarbon group. Examples of the monocyclic hydrocarbon group preferably include a cycloalkyl group having 3 or more and 8 or less carbon atoms, such as a cyclopropyl group, a cyclobutyl group, a cyclopentyl group, a cyclohexyl group, or a cyclooctyl group. Examples of the polycyclic hydrocarbon group preferably include a cycloalkyl group having 6 or more and 20 or less carbon atoms, such as an adamantyl group, a norbornyl group, an isobornyl group, a camphanyl group, a dicyclopentyl group, an α-pinyl group, a tricyclodecanyl group, a tetracyclododecyl group, or an androstanyl group. The cycloalkyl group has carbon atoms forming a cycloalkane ring in which a carbon atom other than the carbon atom at the 1-position may be substituted with a heteroatom such as an oxygen atom. In this case, the number of carbon atoms to be substituted with the heteroatom is not particularly limited.

Examples of the aryl group represented by each of R^{I} to R^{VI} preferably include an aryl group having 6 or more and 14 or less carbon atoms, such as a phenyl group, a naphthyl group, or an anthryl group.

Examples of the aralkyl group represented by each of R^{I} to R^{VI} preferably include an aralkyl group having 7 or more and 12 or less carbon atoms, such as a benzyl group, a phenethyl group, or a naphthylmethyl group.

Examples of the alkenyl group represented by each of R^{I} to R^{VI} preferably include an alkenyl group having 2 or more and 8 or less carbon atoms, such as a vinyl group, an allyl group, a butenyl group, and a cyclohexenyl group.

The ring formed by at least two of R^{I} to R^{III} being bonded to each other and the ring formed by R^{IV} and R^{V} being bonded to each other is each preferably a cycloalkane ring. Examples of the cycloalkane ring preferably include a monocyclic cycloalkane ring such as a cyclopropane ring, a cyclobutane ring, a cyclopentane ring, or a cyclohexane ring; and a polycyclic cycloalkane ring such as a norbornane ring, a tricyclodecane ring, a tetracyclododecane ring, or an adamantane ring.

The alkyl group, cycloalkyl group, aryl group, aralkyl group, and alkenyl group represented by each of R^{I} to R^{VI}, and the cycloalkane ring may each have a substituent, as long as the substituent does not inhibit the radical polymerization reaction.

In particular, the acid-decomposable group is preferably a tert-butyl group, a tert-amyl group, or a group represented by each of the following formulae (I) to (IV).

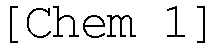

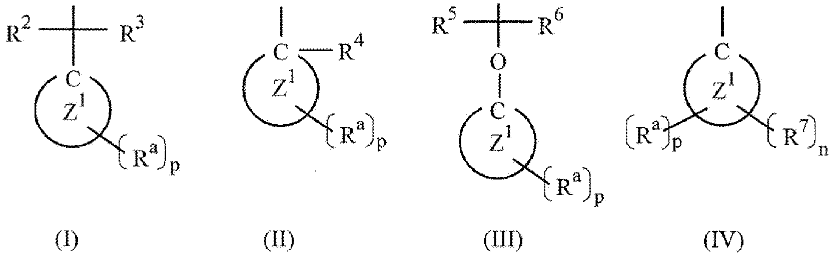

In formulae (I) to (IV), R² to R⁷, R^{a}, n, p, and ring Z¹ respectively have the same meanings as R² to R⁷, R^{a}, n, p, and ring Z¹ in formulae (a1) to (a4) described below.

The acid-decomposable group may be bonded to the radical-polymerizable functional group via a spacer. Examples of such a spacer include the linking group represented by A in formula (1) described below.

Examples of the monomer having an acid-decomposable group include a monomer represented by formula (1) below.

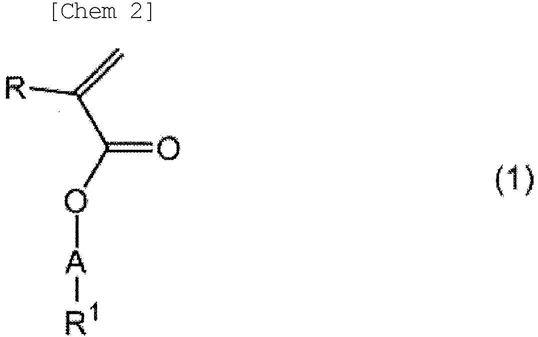

In formula (1), R¹ represents an acid-decomposable group. In formula (1), R represents a hydrogen atom, a halogen atom, or an alkyl group having 1 or more and 6 or less carbon atoms, which may have a halogen atom. Examples of the halogen atom in the alkyl group having 1 or more and 6 or less carbon atoms that may have a halogen atom include a chlorine atom, a bromine atom, and an iodine atom. Examples of the alkyl group having 1 or more and 6 or less carbon atoms in the alkyl group that may have a halogen atom include a methyl group, an ethyl group, a propyl group, an isopropyl group, an n-butyl group, an isobutyl group, a sec-butyl group, a tert-butyl group, a pentyl group, an isoamyl group, a sec-amyl group, a tert-amyl group, and a hexyl group. Examples of the alkyl group having 1 or more and 6 or less carbon atoms that has a halogen atom include a trifluoromethyl group and a 2,2,2-trifluoroethyl group.

In formula (1), A represents a single bond or a linking group. Examples of the linking group include a carbonyl group (-C(=O)-), an ether bond (-O-), an ester bond (-C(=O)-O-), an amide bond (-C(=O)-NH-), a carbonate bond (-O-C(=O)-O-), a group in which a plurality of these linking groups are connected, and a group in which an alkylene group is bonded to any of these linking groups. Examples of the alkylene group include a linear or branched alkylene group such as a methylene group, a methylmethylene group, a dimethylmethylene group, an ethylene group, a propylene group, and a trimethylene group; and a divalent alicyclic hydrocarbon group (particularly, a divalent cycloalkylene group) such as a 1,2-cyclopentylene group, a 1,3-cyclopentylene group, a cyclopentylidene group, a 1,2-cyclohexylene group, a 1,3-cyclohexylene group, a 1,4-cyclohexylene group, and a cyclohexylidene group.

Examples of the monomer represented by formula (1) preferably include one or more monomers selected from the group consisting of the monomers represented by the following formulae (a1) to (a4). Hereinafter, "one or more monomers selected from the group consisting of the monomers represented by formulae (a1) to (a4)" may be referred to as "monomer a".

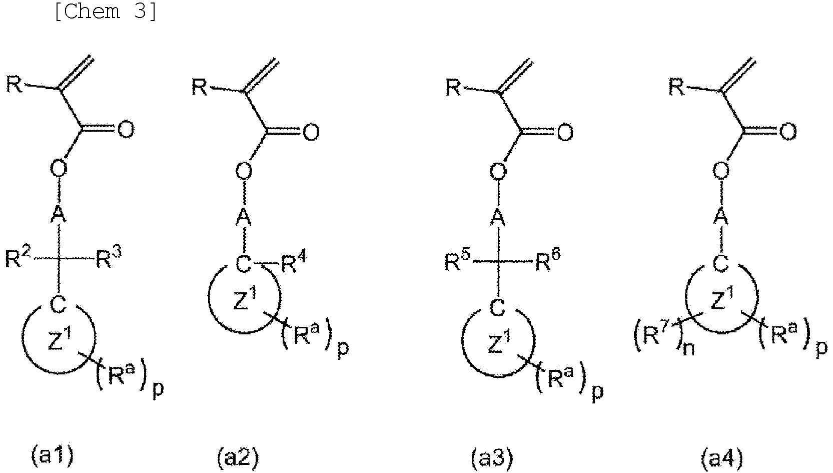

In formulae (a1) to (a4), R represents, as with R in formula (1), a hydrogen atom, a halogen atom, or an alkyl group having 1 or more and 6 or less carbon atoms, which may have a halogen atom, and A represents a single bond or a linking group. A in formulae (a1) to (a4) is preferably a single bond or a group in which an alkylene group is bonded to a carbonyloxy group (an alkylene-carbonyloxy group). R² to R⁴ each independently represent an alkyl group having 1 or more and 6 or less carbon atoms, which may have a substituent. R² and R³ may be bonded to each other to form a ring. R⁵ and R⁶ each independently represent a hydrogen atom or an alkyl group having 1 or more and 6 or less carbon atoms, which may have a substituent. R⁷ represents a -COOR^{c} group. R^{c} represents a tertiary hydrocarbon group, a tetrahydrofuranyl group, a tetrahydropyranyl group, or an oxepanyl group, each of which may have a substituent. n represents an integer of 1 or more and 3 or less. When n is 2 or 3, two or three R⁷s may be the same as or different from each other. R^{a} represents a substituent bonded to the ring Z¹ and independently represents an oxo group, an alkyl group, a hydroxy group that may be protected with a protecting group, a hydroxyalkyl group that may be protected with a protecting group, or a carboxyl group that may be protected with a protecting group. p represents an integer of 0 or more and 3 or less. Z¹ represents an alicyclic hydrocarbon ring having 3 to 20 carbon atoms. When p is 2 or 3, two or three R^{a}s may be the same as or different from each other.

Examples of the alkyl group represented by R^{a} include an alkyl group having 1 or more and 6 or less carbon atoms, such as methyl group, an ethyl group, a propyl group, an isopropyl group, an n-butyl group, a sec-butyl group, a tert-butyl group, a pentyl group, an isoamyl group, a sec-amyl group, a tert-amyl group, and an n-hexyl group.

Examples of the hydroxyalkyl group represented by R^{a} include a hydroxyalkyl group having 1 or more and 6 or less carbon atoms, such as a hydroxymethyl group, a 2-hydroxyethyl group, a 1-hydroxyethyl group, a 3-hydroxypropyl group, a 2-hydroxypropyl group, a 4-hydroxybutyl group, or a 6-hydroxyhexyl group.

Examples of the protecting group that the hydroxy group and the hydroxyalkyl group represented by R^{a} may have include an alkyl group having 1 or more and 4 or less carbon atoms, such as a methyl group, an ethyl group, or a tert-butyl group; a group that forms an acetal bond together with the oxygen atom constituting the hydroxy group (for example, a C₁₋₄ alkyl-O-C₁₋₄ alkyl group such as a methoxymethyl group); and a group that form an ester bond together with the oxygen atom constituting the hydroxy group (for example, an acetyl group and a benzoyl group).

Examples of the protecting group of the carboxy group represented by R^{a} include an alkyl group having 1 or more and 6 or less carbon atoms, such as a methyl group, an ethyl group, a propyl group, an isopropyl group, an n-butyl group, a sec-butyl group, a tert-butyl group, a pentyl group, an isoamyl group, a sec-amyl group, a tert-amyl group, or a hexyl group; a 2-tetrahydrofuranyl group; a 2-tetrahydropyranyl group; and a 2-oxepanyl group.

Examples of the alkyl group having 1 or more and 6 or less carbon atoms represented by R² to R⁶ include a linear or branched alkyl group such as a methyl group, an ethyl group, a propyl group, an isopropyl group, an n-butyl group, an isobutyl group, a sec-butyl group, a tert-butyl group, a pentyl group, an isoamyl group, a sec-amyl group, a tert-amyl group, and a hexyl group. The number of carbon atoms in the alkyl group having 1 or more and 6 or less carbon atoms in R² to R⁶ is preferably 1 or more and 4 or less, more preferably 1 or more and 3 or less, and still more preferably 1 or more and 2 or less.

Examples of the substituent that the alkyl group having 1 or more and 6 or less carbon atoms represented by each of R² to R⁶ may have include a halogen atom, a hydroxy group, a substituted hydroxy group (for example, an alkoxy group having 1 or more and 4 or less carbon atoms, such as a methoxy group, an ethoxy group, or a propoxy group), and a cyano group. Examples of the alkyl group having 1 or more and 6 or less carbon atoms that has a substituent include a haloalkyl group having 1 or more and 6 or less carbon atoms such as a trifluoromethyl group or a 2,2,2-trifluoroethyl group; a hydroxyalkyl group having 1 or more and 6 or less carbon atoms such as a hydroxymethyl group or a 2-hydroxyethyl group; an alkoxyalkyl group having 1 or more and 6 or less carbon atoms such as a methoxymethyl group, a 2-methoxyethyl group, an ethoxymethyl group, or a 2-ethoxyethyl group; and a cyanoalkyl group having 1 or more and 6 or less carbon atoms such as a cyanomethyl group and a 2-cyanoethyl group.

When R² and R³ are bonded to each other to form a ring, examples of the ring include an alicyclic hydrocarbon ring having 3 or more and 12 or less carbon atoms, which may have a substituent.

Examples of the tertiary hydrocarbon group represented by R^{c} include a tert-butyl group and a tert-amyl group.

Examples of the substituent that the tertiary hydrocarbon group represented by R^{c} may have include a halogen atom, a hydroxy group, a substituted hydroxy group (for example, an alkoxy group having 1 or more and 4 or less carbon atoms, such as a methoxy group, an ethoxy group, or a propoxy group), and a cyano group.

Examples of the alicyclic hydrocarbon ring having 3 or more and 20 or less carbon atoms represented by Z¹ include a monocyclic alicyclic hydrocarbon ring, a ring including a norbornane ring or a norbornene ring, an adamantane ring, a ring in which a polycyclic aromatic condensed ring is hydrogenated (preferably completely hydrogenated), and a bridged cyclic hydrocarbon ring having 2 to 6 ring systems. Examples of the monocyclic alicyclic hydrocarbon ring include a 3- to 20-membered (preferably 3- to 15-membered, particularly preferably 5- to 12-membered) cycloalkane ring, such as a cyclopropane ring, a cyclobutane ring, a cyclopentane ring, a cyclohexane ring, or a cyclooctane ring; and a 3- to 20-membered (preferably 3- to 15-membered, particularly preferably 5- to 12-membered) cycloalkene ring, such as a cyclopropene ring, a cyclobutene ring, a cyclopentene ring, or a cyclohexene ring. Examples of the ring including a norbornane ring or a norbornene ring include a norbornane ring, a norbornene ring, a bornane ring, an isobornane ring, a tricyclo[5.2.1.0^{2,6}]decane ring, and a tetracyclo[4.4.0.1^{2,5}.1^{7,10}]dodecane ring. Examples of the ring in which a polycyclic aromatic condensed ring is hydrogenated include a perhydroindane ring, a decalin ring, a tricyclo[7.4.0.0^{3,8}]tridecane ring, and a perhydroanthracene ring. Examples of the bridged clyclic hydrocarbon ring having 2 to 6 ring systems include a tricyclo[4.2.2.1^{2,5}]undecane ring. The number of carbon atoms in the bridged cyclic hydrocarbon ring having 2 to 6 ring systems is preferably 6 or more and 20 or less.

The monomer preferably includes an alicyclic monomer having [-C(=O)-O-], [-S(=O)₂-O-], or [-C(=O)-O-C(=O)-]. When an alicyclic monomer is used, higher substrate adhesiveness and etching resistance can be imparted to the polymer for a photoresist. Hereinafter, the "alicyclic monomer having [-C(=O)-O-], [-S(=O)₂-O-], or [-C(=O)-O-C(=O)-]" may be referred to as "monomer b".

In particular, the monomer b preferably includes one or more monomers selected from the group consisting of monomers represented by formulas (b1) to (b5) below. In formulae (b1) to (b5), R represents a hydrogen atom, a halogen atom, or an alkyl having 1 or more and 6 or less carbon atoms, which may have a halogen atom, and A represents a single bond or a linking group. X represents a single bond, a methylene group, an ethylene group, an oxygen atom, or a sulfur atom. Y represents a methylene group or a carbonyl group. Z represents a divalent organic group (for example, an alkylene group that may be included as A in formulae (a1) to (a4) and that has been illustrated and described as an alkylene group, (particularly a linear alkylene having 1 or more and 3 or less carbon atoms)). V¹ to V³ each independently represent -CH₂-, [-C(=O)-], or [-C(=O)-O-], provided that at least one of V¹ to V³ is [-C(=O)-O-]. R⁸ to R¹⁴ each independently represent a hydrogen atom, a fluorine atom, an alkyl group that may have a fluorine atom, a hydroxy group that may be protected with a protecting group, a hydroxyalkyl group that may be protected with a protecting group, a carboxyl group that may be protected with a protecting group, or a cyano group.

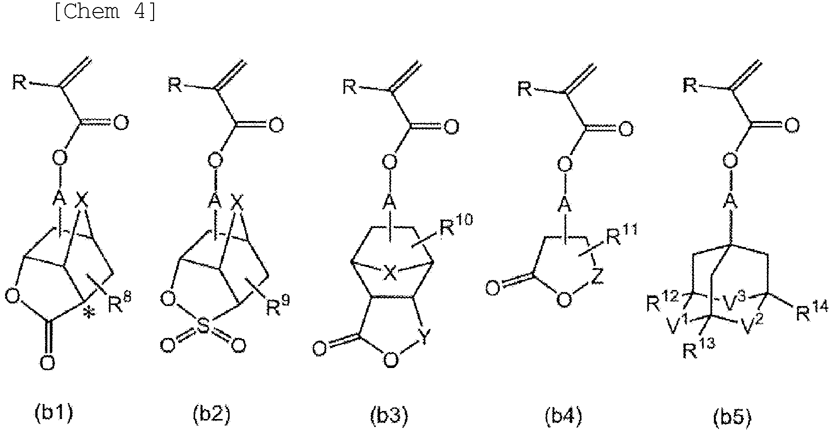

In formulae (b1) to (b5), R and A have the same meanings as R and A in formulae (a1) to (a4), respectively.

In formulae (b1) to (b5), R⁸ to R¹⁴ each represent an alkyl group, a hydroxy group that may be protected with a protecting group, a hydroxyalkyl group that may be protected with a protecting group, or a carboxyl group that may be protected with a protecting group. The alkyl group, the hydroxy group that may be protected with a protecting group, the hydroxyalkyl group that may be protected with a protecting group, or the carboxyl group that may be protected with a protecting group represented by R⁸ to R¹⁴ include groups similar to the alkyl group, the hydroxy group that may be protected with a protecting group, the hydroxyalkyl group that may be protected with a protecting group, and the carboxyl group that may be protected with a protecting group represented by R^{a} in formulae (a1) to (a4). In addition, examples of the alkyl group represented by each of R⁸ to R¹⁴ include a haloalkyl group having 1 or more and 6 or less carbon atoms, such as a trifluoromethyl group or a 2,2,2-trifluoroethyl group.

The monomers represented by formulae (b1) to (b4) may each have one or more of R⁸ to R¹¹, and preferably have one to three of R⁸ to R¹¹. In addition, when the monomers represented by formulae (b1) to (b4) have two or more of R⁸ to R¹¹, the two or more of R⁸ to R¹¹ may be the same as or different from each other.

Among these, the monomer b is preferably a monomer represented by formula (b1) in which R⁸ is an electron-withdrawing group such as a cyano group, a group having an amide group, a group having an imide group, or a fluoroalkyl group having 1 or more and 6 or less carbon atoms; a monomer represented by formula (b2); a monomer represented by formula (b3) in which Y is a carbonyl group; a monomer represented by formula (b4); or a monomer represented by formula (b5). The polymer for a photoresist obtained by polymerization of the monomers exhibits excellent substrate adhesiveness and etching resistance, and also excellent solubility in an alkaline developer, thereby enabling highly accurate formation of fine patterns.

In formula (b1), when R⁸ is an electron-withdrawing group such as a cyano group, a group having an amide group, a group having an imide group, or a fluoroalkyl group having 1 or more and 6 or less carbon atoms, R⁸ is particularly preferably bonded to at least the carbon atom marked with * in formula (b1).

The monomer may further include a monomer c. The monomer c is a monomer represented by formula (c1) below. The polymer for a photoresist obtained by polymerization of a monomer including a monomer c has high transparency and excellent etching resistance. In the formula, R represents a hydrogen atom, a halogen atom, or an alkyl group having 1 or more and 6 or less carbon atoms, which may have a halogen atom. A represents a single bond or a linking group. R^{b} represents a hydroxy group that may be protected with a protecting group, a hydroxyalkyl group that may be protected with a protecting group, a carboxyl group that may be protected with a protecting group, or a cyano group, and is preferably a hydroxy group or a cyano group. q represents an integer of 1 or more and 5 or less. Z² represents an alicyclic hydrocarbon ring having 6 or more and 20 or less carbon atoms. When q is an integer of 2 or more and 5 or less, two to five R^{b}s may be the same as or different from each other.

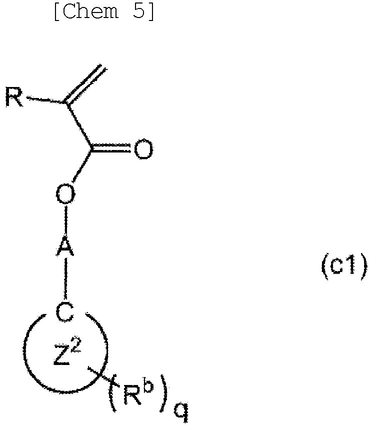

In formula (c1), R and A have the same meanings as R and A in formulae (a1) to (a4), respectively.

In formulae (c1), R^{b} represents a hydroxy group that may be protected with a protecting group, a hydroxyalkyl group that may be protected with a protecting group, or a carboxyl group that may be protected with a protecting group. The hydroxy group that may be protected with a protecting group, the hydroxyalkyl group that may be protected with a protecting group, or the carboxyl group that may be protected with a protecting group represented by R^{b} include groups similar to the hydroxy group that may be protected with a protecting group, the hydroxyalkyl group that may be protected with a protecting group, and the carboxyl group that may be protected with a protecting group represented by R^{a} in formulae (a1) to (a4).

In formula (c1), Z² represents an alicyclic hydrocarbon ring having 6 or more and 20 or less carbon atoms. Examples of the alicyclic hydrocarbon ring having 6 or more and 20 or less carbon atoms include a monocyclic alicyclic hydrocarbon ring, a ring including a norbornane ring or a norbornene ring, an adamantane ring, a ring in which a polycyclic aromatic condensed ring is hydrogenated (preferably completely hydrogenated), and a bridged cyclic hydrocarbon ring having 2 to 6 ring systems. Examples of the monocyclic alicyclic hydrocarbon ring include a 6- to 20-membered (preferably 6- to 15-membered, particularly preferably 6- to 12-membered) cycloalkane ring, such as a cyclohexane ring or a cyclooctane ring; and a 6- to 20-membered (preferably 6- to 15-membered, particularly preferably 6- to 10-membered) cycloalkene ring, such as a cyclohexene ring. Examples of the ring including a norbornane ring or a norbornene ring include a norbornane ring, a norbornene ring, a bornane ring, an isobornane ring, a tricyclo[5.2.1.0^{2,6}]decane ring, and a tetracyclo[4.4.0.1^{2,5}.1^{7,10}]dodecane ring. Examples of the ring in which a polycyclic aromatic condensed ring is hydrogenated include a perhydroindane ring, a decalin ring, a tricyclo[7.4.0.0^{3,8}]tridecane ring, and a perhydroanthracene ring. Examples of the bridged cyclic hydrocarbon ring having 2 to 6 ring systems include a tricyclo[4.2.2.1^{2,5}]undecane ring. The number of carbon atoms in the bridged cyclic hydrocarbon ring having 2 to 6 ring systems is preferably 6 or more and 20 or less. Among these, Z² is preferably a ring including a norbornane ring or a norbornene ring, or an adamantane ring.

### (Radical polymerization initiator)

As the radical polymerization initiator, any known or conventional radical polymerization initiator can be used. Examples of the known or conventional radical polymerization initiator include a radical polymerization initiator containing a cyano group and a radical polymerization initiator containing no cyano group. The radical polymerization initiator may be used singly, or two or more types may be used in combination in any ratio.

Examples of the radical polymerization initiator containing a cyano group include an azo compound containing a cyano group, such as dimethyl 2,2'-azobis(2-methylpropionate), 2,2'-azobis(isobutyronitrile), 2,2'-azobis(2,4-dimethylvaleronitrile), 2,2'-azobis(2-methylbutyronitrile), or 4,4'-azobis(4-cyanovaleric acid).

Examples of the radical polymerization initiator containing no cyano group include an azo compound containing no cyano group, a peroxide-based compound containing no cyano a cyano group, and a redox-based compound containing no cyano group.

Examples of the azo compound containing no cyano group include dimethyl 2,2'-azobisisobutyrate, 2,2'-azobis(2,4,4-trimethylpentane), 2,2'-azobis(2-methylpropane), and dibutyl 2,2'-azobisisobutyrate.

Examples of the peroxide-based compounds containing no cyano group include a ketone peroxide such as methyl ethyl ketone peroxide or cyclohexanone peroxide; a peroxy ketal such as 1,1-bis(tert-hexylperoxy)-3,3,5-trimethylcyclohexane or 1,1-bis(tert-hexylperoxy)cyclohexane; a hydroperoxide such as p-menthane hydroperoxide; a dialkyl peroxide such as 2,5-dimethyl-2,5-bis(tert-butylperoxy) hexane; a diacyl peroxide such as isobutyryl peroxide or 3,3,5-trimethylhexanoyl peroxide; a peroxyester such as 1,1,3,3-tetramethylbutyl peroxyneodecanoate or tert-hexyl peroxyneodecanoate; and a peroxydicarbonate such as di-n-propyl peroxydicarbonate or diisopropyl peroxydicarbonate.

Examples of the redox-based compound containing no cyano group include hydrogen peroxide and ammonium persulfate.

### (Solvent)

Examples of the solvent include a glycol-based solvent (glycol-based compound), an ester-based solvent, a ketone-based solvent, an ether-based solvent, an amide-based solvent, a sulfoxide-based solvent, and a hydrocarbon-based solvent. The solvent may be a single type of solvent or may be a mixed solvent resulting from mixing two or more types in any ratio.

Examples of the glycol-based solvent include propylene glycol monomethyl ether, propylene glycol monomethyl ether acetate, ethylene glycol monomethyl ether acetate, ethylene glycol monoethyl ether acetate, and ethylene glycol monobutyl ether acetate.

Examples of the ester-based solvent include a lactate ester-based solvent such as ethyl lactate; a propionate ester-based solvent such as methyl 3-methoxypropionate; and an acetate ester-based solvent such as methyl acetate, ethyl acetate, propyl acetate, or butyl acetate.

Examples of the ketone-based solvent include acetone, methyl ethyl ketone, methyl isobutyl ketone, methyl amyl ketone, cyclopentanone, and cyclohexanone.

Examples of the ether-based solvent include a chain ether such as diethyl ether, diisopropyl ether, dibutyl ether, or dimethoxyethane; and a cyclic ether such as tetrahydrofuran or dioxane.

Examples of the amide-based solvent include N,N-dimethylformamide.

Examples of the sulfoxide-based solvent includes dimethyl sulfoxide.

Examples of the hydrocarbon-based solvent include an aliphatic hydrocarbon such as pentane, hexane, heptane, or octane; an alicyclic hydrocarbon such as cyclohexane or methylcyclohexane; and an aromatic hydrocarbon, such as benzene, toluene, or xylene.

Among them, the solvent is preferably a glycol-based solvent, such as propylene glycol monomethyl ether or propylene glycol monomethyl ether acetate; an ester-based solvent, such as ethyl lactate; a ketone-based solvent, such as acetone, methyl ethyl ketone, methyl isobutyl ketone, methyl amyl ketone, cyclopentanone, or cyclohexanone; or a mixed solvent thereof.

### (Chain transfer agent)

As the chain transfer agent, a known or conventional chain transfer agent used in radical polymerization can be used. Examples of the known or conventional chain transfer agent include a chain transfer agent containing a mercapto group, and a chain transfer agent containing a thiocarbonylthio group (a chain transfer agent containing a cyano group and a thiocarbonylthio group, or a chain transfer agent ccontaining no cyano group and containing a thiocarbonylthio group). The chain transfer agent may be used singly, or two or more types may be used in combination in any ratio.

Examples of the chain transfer agent containing a mercapto group include a thiol such as 1-butanethiol, 2-butanethiol, tert-butyl mercaptan, 2-methyl-1-propanethiol, 2-methyl-2-propanethiol, 1-octanethiol, 1-decanethiol, 1-dodecanethiol, 1-tetradecanethiol, n-lauryl mercaptan, cyclohexanethiol, 1-mercaptoethanol, 2-mercaptoethanol, 3-mercapto-1-propanol, 3-mercapto-1,2-propanediol, triethylene glycol dimercaptan, p-mercaptophenyl methanol, 2-(p-mercaptophenyl) ethanol, p-(mercaptomethyl)phenyl methanol, 2-(p-(mercaptomethyl) phenyl) ethanol, p-mercaptophenol, p-(mercaptomethyl)phenol, p-(1-mercaptoethyl)phenol, or p-(2-mercaptoethyl)phenol (preferably a thiol having an aliphatic hydrocarbon group having 1 or more and 20 or less carbon atoms, which may have a substituent, and more preferably a thiol having an aliphatic hydrocarbon group having 6 or more and 12 or less carbon atoms, which may have a substituent); a thiol acid such as mercaptopropionic acid, thiobenzoic acid, thioglycolic acid, or thiomalic acid; and a thiol acid ester such as methyl thioglycolate, ethyl thioglycolate, n-butyl thioglycolate, methyl 2-mercaptopropionate, ethyl 2-mercaptopropionate, methyl 3-mercaptopropionate, ethyl 3-mercaptopropionate, methyl p-mercaptobenzoate, ethyl p-mercaptobenzoate, methyl p-(mercaptomethyl)benzoate, or ethyl p-(mercaptomethyl)benzoate (preferably a thiol acid alkyl ester).

Examples of the chain transfer agent containing a cyano group and a thiocarbonylthio group include a dithiobenzoate-based chain transfer agent containing a cyano group, such as 2-cyano-2-propyl 4-cyanobenzodithioate, 4-cyano-4-(phenylcarbonothioylthio)pentanoic acid, 2-cyano-2-propylbenzodithioate, or 4-cyano-4-(phenylcarbonothioylthio)pentanoic acid N-succinimidyl ester; a trithiocarbonate-based chain transfer agent containing a cyano group, such as 4-cyano-4-[(dodecylsulfanylthiocarbonyl)sulfanyl]pentanoic acid, 2-cyano-2-propyl dodecyl trithiocarbonate, 4-cyano-4-[(dodecylsulfanylthiocarbonyl)sulfanyl]pentanol, poly(ethylene glycol)methyl ether 4-cyano-4-[(dodecylsulfanylthiocarbonyl)sulfanyl]pentanoate, poly(ethylene glycol)methyl ether(4-cyano-4-pentanoate dodecyl trithiocarbonate), poly(ethylene glycol)methyl ether(4-cyano-4-pentanoate dodecyl trithiocarbonate), poly(ethylene glycol)methyl ether(4-cyano-4-pentanoate dodecyl trithiocarbonate), or cyanomethyl dodecyl trithiocarbonate; a dithiocarbamate-based chain transfer agent containing a cyano group, such as cyanomethyl methyl(phenyl)carbamodithioate, cyanomethyl diphenylcarbamodithioate, 1-succinimidyl-4-cyano-4-[N-methyl-N-(4-pyridyl)carbamothioylthio]pentanoate, 2-cyanopropan-2-yl N-methyl-N-(pyridin-4-yl)carbamodithioate, or cyanomethyl methyl(4-pyridyl)carbamodithioate; and a xanthate-based chain transfer agent containing a cyano group. Among them, from viewpoint of obtaining a polymer having a smaller polydispersity index Mw/Mn, the chain transfer agent containing a cyano group and a thiocarbonylthio group is preferably 4-cyano-4-(phenylcarbonothioylthio)pentanoic acid, 2-cyano-2-propylbenzodithioate, 4-cyano-4-[(dodecylsulfanylthiocarbonyl)sulfanyl]pentanoic acid, or 2-cyano-2-propyl dodecyl trithiocarbonate.

Examples of the chain transfer agent containing no cyano group and containing a thiocarbonylthio group include a dithiobenzoate-based chain transfer agent containing no cyano group, such as 2-phenyl-2-propyl benzodithioate, 1-(methoxycarbonyl)ethyl benzodithioate, benzyl benzodithioate, ethyl 2-methyl-2-(phenylthiocarbonylthio)propionate, methyl 2-phenyl-2-(phenylcarbonothioylthio)acetate, ethyl 2-(phenylcarbonothioylthio)propionate, or bis(thiobenzoyl)disulfide; a trithiocarbonate-based chain transfer agent containing no cyano group, such as 2-(dodecylthiocarbonylthioylthio)propionic acid, 2-(dodecylthiocarbonylthioylthio)-2-methylpropionic acid, methyl 2-(dodecylthiocarbonylthioylthio)-2-methylpropionate, 2-(dodecylthiocarbonylthioylthio)-2-methylpropionic acid N-hydroxysuccinimide ester, poly(ethylene glycol) methyl ether (2-methyl-2-propionic acid dodecyl trithiocarbonate), poly(ethylene glycol) bis[2-(dodecylthiocarbonylthioylthio)-2-methylpropionate], 2-(dodecylthiocarbonylthioylthio)-2-methylpropionic acid 3-azido-1-propanol ester, 2-(dodecylthiocarbonylthioylthio)-2-methylpropionic acid pentafluorophenyl ester, poly(ethylene glycol) methyl ether 2-(dodecylthiocarbonylthioylthio)-2-methylpropionate, poly(ethylene glycol) methyl ether 2-(dodecylthiocarbonylthioylthio)-2-methylpropionate, poly(ethylene glycol) methyl ether 2-(dodecylthiocarbonylthioylthio)-2-methylpropionate, poly(ethylene glycol) bis[2-(dodecylthiocarbonylthioylthio)-2-methylpropionate], or bis(dodecylsulfanylthiocarbonyl)disulfide; a dithiocarbamate-based chain transfer agent containing no cyano group, such as benzyl 1H-pyrrole-1-carbodithioate, methyl 2-propionate methyl(4-pyridyl)carbamodithioate, or N,N'-dimethyl-N,N'-di(4-pyridyl)thiuram disulfide; and a xanthate-based chain transfer agent containing no cyano group. Among these, in terms of obtaining a polymer having a smaller polydispersity index Mw/Mn, the chain transfer agent containing no cyano group and containing a thiocarbonylthio group is preferably ethyl 2-methyl-2-(phenylthiocarbonylthio)propionate.

Although the radical polymerization reaction of a monomer using a microfluidic system has been described above, the polymerization reaction when polymerizing a monomer by the microfluidic system according to the present embodiment is not limited to a radical polymerization reaction, and a known or conventional polymerization reaction may be appropriately adopted. In addition, as described above, the fact that uniform progress of a chemical reaction becomes possible because the components in the reaction system are uniformly mixed is not limited to the polymerization reaction of a monomer, and the same applies to various chemical reactions. Therefore, even when the microfluidic system according to the present embodiment is used for a chemical reaction other than the polymerization reaction of a monomer, the chemical reaction can proceed uniformly, and consequently, it goes without saying that the quality of the product can be ensured.

### [Polymer production system]

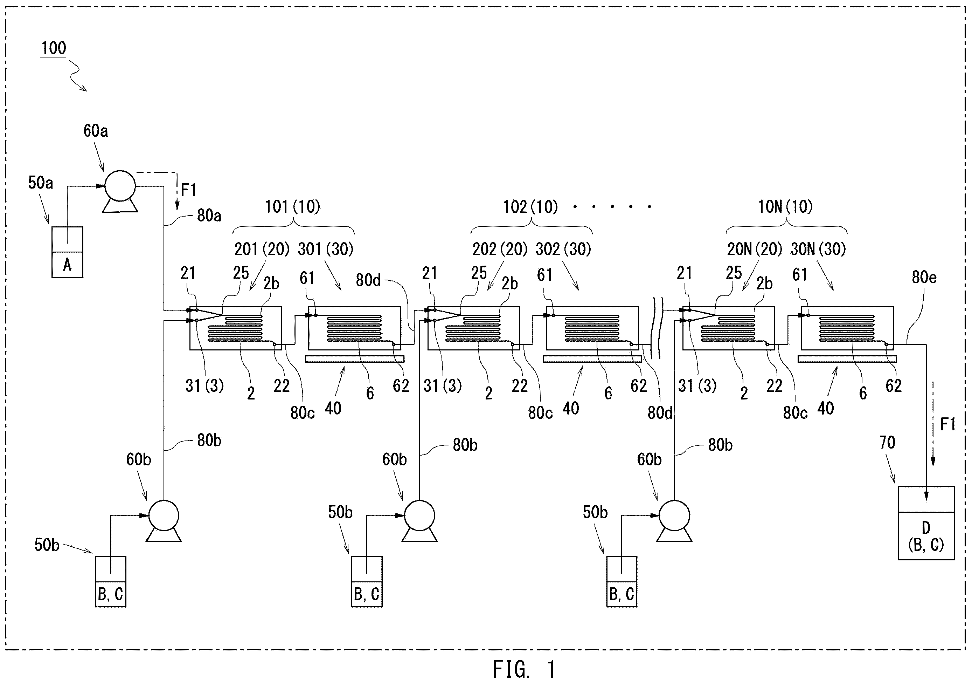

Hereinafter, a polymer production system, which is an example of the microfluidic system according to the present disclosure, is described with reference to the drawings. Fig. 1 is a configuration diagram of a polymer production system 100 according to the present embodiment. In the present embodiment, a polymer production system 100 for producing a polymer D is described as an example of the microfluidic system according to the present disclosure, the polymer D being a reaction product formed by polymerizing two monomers, a monomer B and a monomer C, in the presence of a polymerization initiator A. However, the configuration of the embodiment described below is merely illustrative, and the technology of the present disclosure is not limited to the configuration of this embodiment. In the present specification, "immediately upstream" refers to the upstream side of two adjacent components in the flow direction, and "immediately downstream" refers to the downstream side of two adjacent components in the flow direction.

### [Overall configuration]

First, an overall configuration of the polymer production system 100 is described. The polymer production system 100 is configured to include multiple chip-type microfluidic devices in which microchannels are formed on a substrate. More specifically, as illustrated in Fig. 1, the polymer production system 100 includes a plurality of device units 10 that include a mixing device 20 and a reaction device 30, each of which is a chip-type microfluidic device, a heater 40, raw material tanks 50a and 50b, liquid-feeding pumps 60a and 60b, a product tank 70, and conduits 80a, 80b, 80c, 80d, and 80e. In the present embodiment, the "upstream side" refers to the side of the raw material tank 50a in a production flow channel F1 (an example of a "predetermined processing channel") extending from the raw material tank 50a to the product tank 70, and the "downstream side" refers to the side of the product tank 70 in the production flow channel Fl. Note that the production flow channel F1 is a continuous flow channel extending from the raw material tank 50a, which serves as a fluid supply source, to the product tank 70, which serves as a fluid discharge destination.

The raw material tank 50a stores a solution containing a radical polymerization initiator A (polymerization initiator A solution). The radical polymerization initiator A (hereinafter sometimes simply referred to as a "polymerization initiator A") generates radicals when subjected to treatment such as heating or light irradiation, thereby promoting the polymerization reaction of a curable compound. The raw material tank 50b stores a solution containing a monomer B and a monomer C as reactants (monomer BC solution). The monomer B and the monomer C undergo radical polymerization with each other in the presence of the polymerization initiator A. The monomer B and the monomer C may be different types of monomers, in which case copolymerization occurs. Alternatively, the monomer B and the monomer C may be the same type of monomer, in which case homopolymerization occurs. The polymerization initiator A solution and the monomer BC solution are each an example of the "plurality of different fluids" according to the present disclosure.

As illustrated in Fig. 1, in the polymer production system 100, a plurality of mixing devices 20 and a plurality of reaction devices 30 are arranged alternately toward the downstream side in the flow direction, with one of the mixing devices 20 positioned at the most upstream (head). A mixing device 20 and a reaction device 30 that are adjacent to each other in the flow direction are connected by conduits 80d and 80e to enable fluid communication with each other. Accordingly, in the polymer production system 100, the plurality of mixing devices 20 and the plurality of reaction devices 30 are arranged alternately in series. A heater 40, which is described later, is also provided together with the reaction device 30.

A device unit 10 is a combination of microfluidic devices composed of a mixing device 20 and a reaction device 30 that are adjacent to each other in the flow direction. In the device unit 10, the mixing device 20 is arranged on the upstream side and the reaction device 30 is arranged on the downstream side. The polymer production system 100 according to the present embodiment is configured to include a plurality of device units 10. Hereinafter, the number of device units 10 in the present embodiment is denoted as N (where N is an integer of 2 or more). The number N of device units 10 is not particularly limited, but is preferably 5 or more, for example. For example, the number N is preferably 3 or more, more preferably 4 or more, and still more preferably 5 or more.

Among the plurality of device units 10 included in the polymer production system 100, the k-th device unit 10 (where k is an integer of 1 or more and N or less) from the upstream side is denoted as device unit 10k. That is, in the polymer production system 100, a device unit 101, a device unit 102, ..., and a device unit 10N are arranged in order from the upstream side. Among the plurality of mixing device 20 included in the polymer production system 100, the mixing device 20 of the k-th device unit 10 from the upstream side is denoted as mixing device 20k. That is, in the polymer production system 100, a mixing device 201, a mixing device 202, ..., and a mixing device 20N are arranged in order from the upstream side. Similarly, among the plurality of reaction device 30 included in the polymer production system 100, the reaction device 30 of the k-th device unit 10 from the upstream side is denoted as reaction device 30k. That is, in the polymer production system 100, a reaction device 301, a reaction device 302, ..., and a reaction device 30N are arranged in order from the upstream side. In the present embodiment, the device unit 10k is configured as a combination of the mixing device 20k and the reaction device 30k.

The polymer production system 100 continuously supplies a polymerization initiator A solution and a monomer BC solution to the production flow channel F1 through the mixing device 201 of the device unit 101 arranged at the most upstream side, and continuously and additionally supplies (additionally charges) the monomer BC solution to the production flow channel F1 through the mixing device 20 of the device unit 10 disposed downstream of the device unit 101, thereby sequentially producing a polymer D. The polymer D produced by the polymer production system 100 is discharged from the reaction device 30N of the device unit 10N positioned at the most downstream among the plurality of device units 10.

The conduits 80a, 80b, 80c, 80d, and 80e are tubes through which a fluid can flow. The conduit 80a connects the raw material tank 50a and the mixing device 201 of the device unit 101 to allow fluid communication therebetween, and the conduit 80b connects the raw material tank 50b and the mixing device 20 to allow fluid communication therebetween. In addition, the conduit 80c connects the mixing device 20 and the reaction device 30 in the device unit 10 to allow fluid communication therebetween. Further, the conduit 80d connects the reaction device 30 of the upstream device unit 10 and the mixing device 20 of the downstream device unit 10 in adjacent device units 10 in the flow direction to allow fluid communication therebetween. The conduit 80e connects the reaction device 30N of the device unit 10N and the product tank 70 to allow fluid communication therebetween.

The raw material tanks 50a and 50b are containers in which the polymerization initiator A solution and the monomer BC solution are respectively accommodated, as described above. The polymerization initiator A solution and the monomer BC solution are respectively supplied to the mixing device 20 through the conduits 80a and 80b. The product tank 70 is a container that stores the polymer D produced by the polymer production system 100. The polymer D is discharged from the reaction device 30N of the device unit 10N to the product tank 70 through the conduit 80e.

The conduits 80a and 80b are provided with liquid-feeding pumps 60a and 60b, respectively. The liquid-feeding pumps 60a and 60b are liquid-feeding units configured to transport the fluids in the raw material tanks 50a and 50b to the mixing device 20. The polymerization initiator A solution in the raw material tank 50a is pressure-fed through the conduit 80a by driving the liquid-feeding pump 60a and supplied to the mixing device 20. The monomer BC solution in the raw material tank 50b is pressure-fed through the conduit 80b by driving the liquid-feeding pump 60b and supplied to the mixing device 20. Examples of the liquid-feeding pump include a syringe pump, a diaphragm pump, and a pressure-controlled pump. In the present embodiment, for example, without using the raw material tanks 50a and 50b, the polymerization initiator A solution and the monomer BC solution may be supplied to the reaction device 30 by using syringe pumps in which the respective solutions are accommodated.

### [Mixing Device]

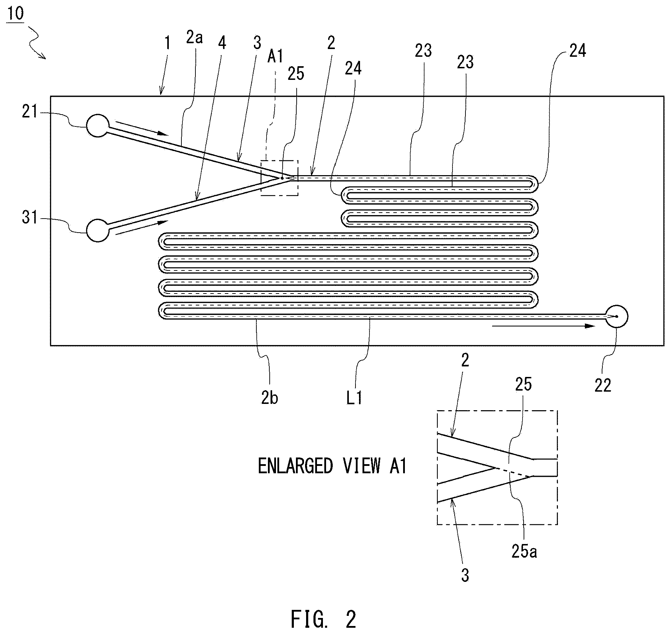

Next, the structure of the mixing device 20 is described. Fig. 2 is a plan diagram of a mixing device 20 according to the present embodiment. The mixing device 20 according to the present embodiment is configured as a chip-type microfluidic device in which a microchannel inside a substrate (also referred to as a microchannel) is formed. As illustrated in Fig. 2, the mixing device 20 has a substrate 1, a first channel 2, and a first introduction channel 3.

The substrate 1 is a plate-like member having a rectangular shape in plan view. The material of the substrate 1 is not particularly limited, and examples thereof include silicon, silica, quartz, glass, resin, and silicon carbide. From the viewpoint of producing a resist polymer, a glass chip-can be suitably used as a non-metallic, solvent-resistant material.

The first channel 2 and the first introduction channel 3 are configured as grooves formed on the surface of the substrate 1. The first channel 2 and the first introduction channel 3 can be formed, for example, by subjecting the substrate 1 to etching treatment.

The first channel 2 is formed as a microchannel that constitutes a part of the production flow channel F1. As illustrated in Fig. 2, the first channel 2 has a meandering shape in plan view. A supply port 21 for supplying a fluid into the first channel 2 is formed at one end of the first channel 2, and a discharge port 22 for discharging a solution from the first channel 2 is formed at the other end of the first channel 2. That is, the first channel 2 extends continuously from the supply port 21 to the discharge port 22 while meandering. The fluid supplied to the supply port 21 flows through the first channel 2 and is discharged from the discharge port 22. The first channel 2 includes a plurality of straight channels 23 that are formed in a linear shape and arranged in parallel in plan view, and a plurality of curved channels 24 that are formed in a curved shape and connect adjacent straight channels 23 and the ends of 23 in the flow direction. The straight channels 23 and the curved channels 24 form the first channel 2 in a meandering shape.

In addition, in an intermediate portion of the first channel 2 in the flow direction, more specifically, in an intermediate portion of the straight channel 23 positioned at the most upstream in the first channel 2, an introduction unit 25 configured to introduce a fluid from the first introduction channel 3 into the first channel 2 is formed. The introduction unit 25 introduces the monomer BC solution into the first channel 2 in order to mix the polymerization initiator A solution and the monomer BC solution, which are a plurality of different fluids, in the production flow channel F1 (in this example, the first channel 2). The introduction unit 25 is formed as a merging portion that merges with the first introduction channel 3. That is, the first channel 2 merges with the first introduction channel 3 at the introduction unit 25. As illustrated in the enlarged view A1 of Fig. 2, the introduction unit 25 has a first introduction port 25a that opens to the first introduction channel 3.

In the present specification, in the mixing device, a microchannel that causes mixing of reactants contained in a mixed fluid of a plurality of different fluids to proceed is referred to as a "mixing channel". The mixing channel is, that is, a region in which the mixing of reactants introduced at the introduction unit proceeds. In the polymer production system 100 according to the present embodiment, a region of the first channel 2 downstream of the introduction unit 25 corresponds to the "mixing channel". A region of the first channel 2 upstream of the introduction unit 25 is defined as a transport channel 2a, and a region downstream of the introduction unit 25 is defined as a mixing channel 2b. That is, the transport channel 2a is a region from the supply port 21 to the introduction unit 25, and the mixing channel 2b is a region from the introduction unit 25 to the discharge port 22. Although details will be described later, in the transport channel 2a of the mixing device 201 positioned at the most upstream among the plurality of mixing devices 20, the polymerization initiator A solution is transported, and in the transport channel 2a of the mixing device 20 disposed downstream of the mixing device 201, a generated liquid (reaction mixture) containing the polymerization initiator A solution and the polymer D is transported. The fluid transported from the transport channel 2a of the first channel 2 and the monomer BC solution transported from the first introduction channel 3 merge at the introduction unit 25 of the first channel 2 and are mixed while flowing through the mixing channel 2b.

In addition, the channel length (the length in the flow direction) of the mixing channel 2b is defined as L1. L1 is, that is, the channel length of the first channel 2 on the downstream side of the introduction unit 25, and can be defined as the channel length from the introduction unit 25 to the discharge port 22. That is, the channel length L1 can be defined as the channel length of the first channel 2 on the downstream side of the position at which the mixing of the monomer BC solution and the polymerization initiator A solution introduced from the introduction unit 25 is started.

Here, among the plurality of mixing devices 20 included in the polymer production system 100, the channel length L1 of the mixing channel 2b in the k-th mixing device 20 from the upstream side is denoted as L1k. That is, in order from the upstream side, the channel lengths L1 of the mixing channels 2b in the mixing device 201, the mixing device 202, ..., and the mixing device 20N become L11, L12, ..., and L1N. At this time, in the polymer production system 100 according to the present embodiment, while the cross-sectional areas of the mixing channels 2b of the plurality of mixing devices 201 to 20N are made equivalent (constant), the channel length L1 of the mixing channel 2b in each mixing device 20 is set such that L1k < L1(k+1). That is, in the plurality of mixing devices 20, the channel length L1 of the mixing channel 2b becomes longer in the mixing devices 20 located further downstream, and the channel length L1 of the mixing channel 2b in each mixing device 20 is set accordingly. Therefore, in the polymer production system 100 according to the present embodiment, the volume of the mixing channel 2b becomes larger in the mixing devices 20 located further downstream. However, the technology of the present disclosure is not limited thereto, and the channel length can be set as appropriate.

The first introduction channel 3 is a channel for introducing the monomer BC solution into the first channel 2. The first introduction channel 3 is formed in a linear shape. At one end of the first introduction channel 3, a supply port 31 for supplying a fluid (in this example, the monomer BC solution) into the first introduction channel 3 is formed, and the other end of the first introduction channel 3 is connected to the introduction unit 25 of the first channel 2. As a result, the first introduction channel 3 and the first channel 2 communicate with each other through the first introduction port 25a. The transport channel 2a of the first channel 2, the first introduction channel 3, and the mixing channel 2b form a substantially Y-shaped configuration. The monomer BC solution supplied to the supply port 31 flows through the first introduction channel 3 and is introduced into the first channel 2 from the first introduction port 25a of the introduction unit 25.

As illustrated in Fig. 1, a conduit 80a for supplying the polymerization initiator A solution from the raw material tank 50a to the first channel 2 is connected to the supply port 21 of the first channel 2 in the mixing device 201, which is positioned at the most upstream among the plurality of mixing devices 20. A conduit 80d for supplying a generated liquid containing the polymerization initiator A solution and the polymer D from the reaction device 30 to the first channel 2 is connected to the supply port 21 of the first channel 2 in the mixing device 20 disposed downstream of the mixing device 201. In addition, a conduit 80c for supplying a mixed liquid (an example of a mixed fluid) of the polymerization initiator A solution and the monomer BC from the first channel 2 to the reaction device 30 is connected to the discharge port 22 of the first channel 2 in the mixing device 20. As a result, the first channel 2 constitutes a part of the production flow channel F1. Furthermore, a conduit 80b for supplying the monomer BC solution from the raw material tank 50b to the first introduction channel 3 is connected to the supply port 31 of the first introduction channel 3 in the mixing device 20.

In the technology according to the present disclosure, the introduction channel (the first introduction channel 3 in this example) for introducing a fluid into the mixing channel is not an essential component. For example, the mixing device 20 may not have the first introduction channel 3 and may adopt a mode in which the monomer BC solution is directly introduced from the introduction unit 25 of the first channel 2. However, from the viewpoint of efficient mixing, it is preferable that the mixing device has an introduction channel that guides a fluid to the introduction unit. In addition, the mixing device may have a plurality of introduction units. For example, the mixing device 20 may introduce a monomer B solution containing monomer B and a monomer C solution containing monomer C into the mixing channel 2b by separate introduction units.

### [Reaction device]

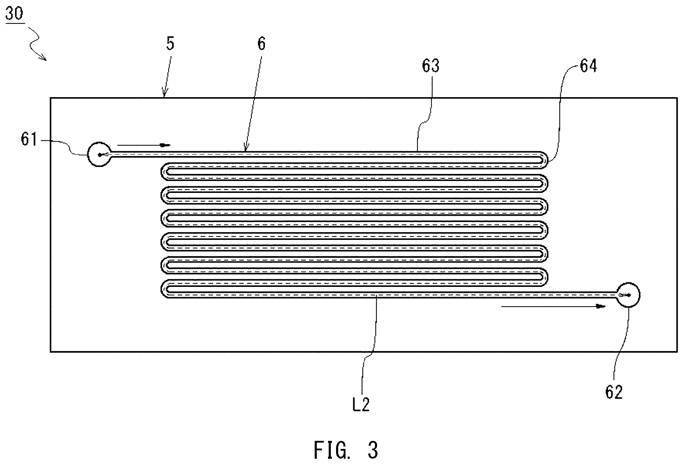

Next, the structure of the reaction device 30 is described. Fig. 3 is a plan diagram of a reaction device 30 according to the present embodiment. The reaction device 30 according to the present embodiment is configured, similarly to the mixing device 20, as a chip-type microfluidic device in which a microchannel is formed inside a substrate. As illustrated in Fig. 3, the reaction device 30 has a substrate 5 and a reaction channel 6. The reaction device 30 is an example of an "other-process device," and a chemical reaction is carried out in the reaction channel 6.

The substrate 5 is a plate-like member having a rectangular shape in plan view. The material of the substrate 5 is not particularly limited, and examples thereof include silicon, silica, quartz, glass, resin, and silicon carbide. From the viewpoint of producing a resist polymer, a glass chip-can be suitably used as a non-metallic, solvent-resistant material.

The reaction channel 6 is configured as a groove formed on the surface of the substrate 5 and can be formed, for example, by performing an etching process on the substrate 5.

The reaction channel 6 is a channel for progressing a chemical reaction of the mixed liquid of the polymerization initiator A solution and the monomer BC solution mixed in the mixing channel 2b of the mixing device 20, and is formed as a microchannel constituting a part of the production flow channel F1. As illustrated in Fig. 3, the reaction channel 6 has a meandering shape in plan view. A supply port 61 for supplying a fluid into the reaction channel 6 is formed at one end of the reaction channel 6, and a discharge port 62 for discharging a solution from the reaction channel 6 is formed at the other end of the reaction channel 6. That is, the reaction channel 6 extends continuously from the supply port 61 to the discharge port 62 while meandering. The fluid supplied to the supply port 61 flows through the reaction channel 6 and is discharged from the discharge port 62. The reaction channel 6 includes a plurality of straight channels 63 that are formed in a linear shape and arranged in parallel in plan view, and a plurality of curved channels 64 that are formed in a curved shape and connect adjacent straight channels 63 and the ends of 63 in the flow direction. The straight channels 63 and the curved channels 64 form the reaction channel 6 in a meandering shape.

Here, the channel length (the length in the flow direction) of the reaction channel 6 is defined as L2. L2 can be defined as the channel length from the supply port 61 to the discharge port 62. The channel length L2 is not particularly limited and can be set as appropriate.

As illustrated in Fig. 1, a conduit 80c for supplying the mixed liquid of the polymerization initiator A solution and the monomer BC solution from the mixing channel 2b of the mixing device 20 to the reaction channel 6 is connected to the supply port 61 of the reaction channel 6 in the reaction device 30. In addition, a conduit 80d for supplying a generated liquid containing the polymerization initiator A solution and the polymer D from the reaction channel 6 to the first channel 2 of the mixing device 20 is connected to the discharge port 62 of the reaction channel 6 in the reaction device 30 disposed upstream of the reaction device 30N, which is positioned at the most downstream among the plurality of reaction devices 30. Furthermore, a conduit 80e for discharging the generated liquid from the reaction channel 6 to the product tank 70 is connected to the discharge port 62 of the reaction channel 6 in the reaction device 30N. As a result, the reaction channel 6 constitutes a part of the production flow channel F1, and the mixing channel 2b and the reaction channel 6 are connected in series.

Furthermore, although details will be described later, the polymerization reaction of monomer B and monomer C in the presence of the polymerization initiator A is promoted by heating the mixed liquid flowing through the reaction channel 6 by the heater 40 so that the mixed liquid becomes equal to or higher than the reaction temperature. That is, the reaction channel 6 is a microchannel for progressing a chemical reaction of the mixed liquid of the polymerization initiator A solution and the monomer BC solution. In addition, in the reaction device according to the present disclosure, one or more introduction units for introducing at least one of the different fluids into the reaction channel may be provided depending on the types, amounts, or introduction timing of the different fluids. In such a case, the reaction channel can be defined as a microchannel disposed downstream of the introduction unit positioned at the most downstream in the reaction device.

### [Heater]

The heater 40 is a device for promoting a chemical reaction (a polymerization reaction in this example) by heating the mixed liquid flowing through the reaction channel 6 of the reaction device 30. The heater 40 is an example of a "reaction-promoting unit" according to the present disclosure. The heating temperature of the heater 40 is not particularly limited and can be set as appropriate according to the reaction temperature. The heater 40 heats the mixed liquid in the reaction channel 6 so that the polymerization reaction of monomer B and monomer C are promoted and the temperature becomes equal to or higher than the reaction temperature of the polymerization reaction of monomer B and monomer C in the presence of the polymerization initiator A. Here, the reaction temperature refers to, for example, the decomposition temperature of the polymerization initiator A. The heater 40 is not particularly limited and may be, for example, a known heater such as an electric heater. Here, in the present embodiment, the heater 40 is provided together with the reaction device 30 so that the chemical reaction of the reactants is promoted in the reaction channel 6. Here, in the present embodiment, the heater 40 is provided only in the reaction device 30 among the mixing device 20 and the reaction device 30 so that the chemical reaction of the reactants is promoted in the reaction channel 6 among the mixing channel 2b and the reaction channel 6. In other words, in the present embodiment, the fluid flowing through the mixing channel 2b is not heated by the heater 40 and is kept below the reaction temperature. Therefore, in the present embodiment, the progression of the chemical reaction of the reactants contained in the fluid flowing through the mixing channel 2b is suppressed in the mixing channel 2b.

### [Method for producing a polymer]

Hereinafter, a method for producing a polymer using the polymer production system 100 according to the embodiment is described. The production of the polymer according to the embodiment is carried out by continuously supplying the polymerization initiator A solution and the monomer BC solution to the production flow channel F1 including the mixing channel 2b and the reaction channel 6. Specifically, the polymerization initiator A solution is continuously supplied to the first channel 2 of the mixing device 201 by the liquid-feeding pump 60a, and the monomer BC solution is continuously supplied to the first channel 2 of each mixing device 20 by the liquid-feeding pump 60b. The flow rate and flow velocity of each solution supplied to the first channel 2 are not particularly limited and can be set as appropriate depending on the purpose.

In the polymer production system 100, a polymer is generated in each reaction device 30 of each device unit 10. Hereinafter, the mixed liquid supplied from the mixing device 20k, which is the mixing device 20 of the k-th device unit 10 from the upstream side, to the reaction device 30k is referred to as the k-th mixed liquid, and the generated liquid supplied from the reaction device 30k to the mixing device 20(k+1) is referred to as the k-th generated liquid.

First, the generation of the polymer in the device unit 101 positioned at the most upstream is described. The polymerization initiator A solution supplied from the raw material tank 50a by the liquid-feeding pump 60a and flowing through the conduit 80a to the mixing device 201 of the device unit 101 is introduced into the first channel 2 through the supply port 21. On the other hand, the monomer BC solution supplied from the raw material tank 50b by the liquid-feeding pump 60b and flowing through the conduit 80b to the mixing device 201 is introduced into the first introduction channel 3 through the supply port 31.

Then, the monomer BC solution transported from the first introduction channel 3 is introduced into the first channel 2 through the introduction unit 25 and merges with the polymerization initiator A solution transported through the transport channel 2a. As a result, the mixing of the polymerization initiator A solution and the monomer BC solution is started at the introduction unit 25. The polymerization initiator A solution and the monomer BC solution are uniformly mixed while flowing through the mixing channel 2b, which is a microchannel, by diffusion mixing using the mixing channel 2b as a mixing field, until reaching the discharge port 22, under the pressure of the liquid-feeding pumps 60a and 60b.

Here, the fluid flowing through the mixing channel 2b of the mixing device 201 is not heated by the heater 40 and is kept below the reaction temperature. Therefore, the polymerization reaction between the monomer B and the monomer C is suppressed from proceeding in the mixing channel 2b.

The primary mixed liquid of the polymerization initiator A solution and the monomer BC solution is discharged from the discharge port 22 and flows through the conduit 80c to be supplied to the reaction device 301 of the device unit 101.

The primary mixed liquid supplied to the reaction device 301 is introduced into the reaction channel 6 through the supply port 61. The primary mixed liquid introduced into the reaction channel 6 flows through the reaction channel 6 while being heated by the heater 40 so as to become equal to or higher than the reaction temperature. As a result, the polymerization reaction between the monomer B and the monomer C is promoted in the presence of the polymerization initiator A to yield a polymer D. At this time, a part of the polymerization initiator A contained in the primary mixed liquid, at least a part of the monomer B, and at least a part of the monomer C are consumed by the polymerization reaction. The monomer B and the monomer C may be fully polymerized, or may be polymerized such that a portion thereof remains unreacted.

The primary generated liquid containing the polymerization initiator A and the polymer D is discharged from the discharge port 62 and flows through the conduit 80d to be supplied to the mixing device 202 of the device unit 102.

Next, the generation of the polymer in the device unit 10p (p is an integer of 2 or more and less than N) disposed downstream of the device unit 101 is described. The (p-1)-th generated liquid supplied from the reaction device 30(p-1) of the device unit 10(p-1), which is disposed immediately upstream of the device unit 10p, flows through the conduit 80d and is supplied to the mixing device 20p of the device unit 10p, and is introduced into the first channel 2 through the supply port 21. The (p-1)-th generated liquid contains the polymerization initiator A and the polymer D. In addition, the (p-1)-th generated liquid may contain monomer B or monomer C that has not reacted in each reaction device 30 located upstream of the mixing device 20p.

On the other hand, similarly to the case of the mixing device 201, the monomer BC solution is supplied to the mixing device 20p by the liquid-feeding pump 60b. Then, the monomer BC solution transported from the first introduction channel 3 is introduced into the first channel 2 through the introduction unit 25 and merges with the (p-1)-th generated liquid transported through the transport channel 2a. As a result, the monomer BC solution is additionally introduced into the production flow channel F1, and an additional charge of the monomer is performed. Then, at the introduction unit 25 of the first channel 2, the mixing of the (p-1)-th generated liquid containing the polymerization initiator A and the polymer D and the additionally introduced monomer BC solution is started. The (p-1)-th generated liquid and the monomer BC solution are uniformly mixed while flowing through the mixing channel 2b under the pressure of the liquid-feeding pumps 60a and 60b, until reaching the discharge port 22.

Here, similarly to the case of the mixing device 201, the fluid flowing through the mixing channel 2b of the mixing device 20p is not heated by the heater 40 and is kept below the reaction temperature. Therefore, the polymerization reaction between the monomer B and the monomer C is suppressed from proceeding in the mixing channel 2b.

The p-th mixed liquid of the (p-1)-th generated liquid and the monomer BC solution is discharged from the discharge port 22 and flows through the conduit 80d to be supplied to the reaction device 30p of the device unit 10p.

The p-th mixed liquid supplied to the reaction device 30p is introduced into the reaction channel 6 through the supply port 61 and flows through the reaction channel 6 while being heated by the heater 40 so as to become equal to or higher than the reaction temperature. As a result, the polymerization reaction between the monomer B and the monomer C is promoted in the presence of the polymerization initiator A to yield a polymer D. At this time, a part of the polymerization initiator A contained in the p-th mixed liquid, at least a part of the monomer B, and at least a part of the monomer C are consumed by the polymerization reaction. The monomer B and the monomer C may be fully polymerized, or may be polymerized such that a portion thereof remains unreacted. In addition, when monomer B or monomer C that has not reacted in each reaction device 30 located upstream of the mixing device 20p remains in the p-th mixed liquid, these may also be polymerized.

The p-th generated liquid containing the polymerization initiator A and the polymer D is discharged from the discharge port 62 and flows through the conduit 80d to be supplied to the mixing device 20(p+1) of the device unit 10(p+1) disposed immediately downstream.

Next, the production of the polymer in the device unit 10N positioned at the most downstream is described. Since the mixing process in the mixing device 20N of the device unit 10N and the chemical reaction in the reaction device 30N are the same as those in the device unit 10p, detailed descriptions thereof are omitted.

Similarly to the mixing device 20p, in the mixing device 20N of the device unit 10N, the (N-1)-th generated liquid containing the polymerization initiator A and the polymer D and the additionally introduced monomer BC solution are uniformly mixed while the progress of the polymerization reaction is suppressed, until reaching the discharge port 22. Then, similarly to the reaction device 30p, in the reaction device 30N of the device unit 10N, the N-th mixed liquid flows through the reaction channel 6 while being heated by the heater 40 so as to become equal to or higher than the reaction temperature, whereby the polymerization reaction is promoted and the polymer D is generated. At this time, in the reaction channel 6 of the reaction device 30N, all of the polymerization initiator A contained in the N-th mixed liquid may be consumed by the polymerization reaction of the monomer B and the monomer C. In addition, all of the monomer B or all of the monomer C contained in the N-th mixed liquid may be consumed by the polymerization reaction.

The N-th generated liquid containing the polymer D is discharged from the discharge port 62 and flows through the conduit 80e to be discharged into the product tank 70. As a result, the polymer D generated in the device units 101, 102, ..., 10N is recovered. As described above, in the method for producing a polymer using the polymer production system 100 according to the present embodiment, by performing (N-1) additional charges, a large amount of the polymer D can be produced.

### [Operation and Effects]

As described above, the polymer production system 100 according to the present embodiment includes a plurality of mixing devices 20 having a mixing channel 2b for mixing the polymerization initiator A solution and the monomer BC solution, which are a plurality of different fluids, and an introduction unit 25 for introducing the monomer BC solution into the mixing channel 2b, and a plurality of reaction devices 30 having a reaction channel 6 for progressing the polymerization reaction of the monomer B and the monomer C (reactants) contained in the mixed liquid mixed in the mixing channel 2b. In the polymer production system 100, the plurality of mixing devices 20 and the plurality of reaction devices 30 are arranged in series so that the mixing channel 2b and the reaction channel 6 communicate with each other.

If the polymer production system 100 does not include the mixing device 20 and performs mixing and reaction only with the reaction device 30, the polymerization initiator A solution, the monomer B solution, and the monomer C solution are not sufficiently uniformly mixed, and the polymerization reaction is carried out in a state where the concentrations of the polymerization initiator A, the monomer B, and the monomer C are uneven in the reaction system. As a result, the polymerization reaction does not proceed uniformly, and problems may occur such as the characteristics of the polymer D, for example, the monomer sequence and the polydispersity Mw/Mn, not becoming the desired ones, and the quality of the reaction product cannot be ensured.

In contrast, the polymer production system 100 according to the present embodiment mixes the polymerization initiator A solution, the monomer B solution, and the monomer C solution in the mixing device 20, and then progresses the polymerization reaction of the monomer B and the monomer C contained in the mixed liquid in the reaction device 30. Therefore, in the reaction device 30, the polymerization reaction is carried out in a state where the polymerization initiator A solution and the monomer BC solution are more uniformly mixed. As a result, according to the polymer production system 100, the polymerization reaction can be progressed more uniformly. Accordingly, for example, it becomes easier to make the characteristics of the polymer D, such as the monomer sequence and the polydispersity Mw/Mn, the desired ones, and the quality of the reaction product can be more easily ensured.

In addition, in order to uniformly mix a plurality of different fluids and uniformly progress chemical, biological, or physical processes in the other process device, the microfluidic system according to the present disclosure only needs to include one or more mixing devices and one or more other process devices, and the number of mixing devices or the number of other process devices does not need to be plural. According to the microfluidic system of the present disclosure, by separating the mixing device in which the mixing process is performed and the other process device in which processes other than the mixing process are performed, the chemical, biological, or physical processes in the other process device can be progressed in a state where the plurality of different fluids are uniformly mixed. In the present embodiment, from the viewpoint of uniformly progressing the chemical reaction in the reaction device 30, it is sufficient that one or more mixing devices 20 and one or more reaction devices 30 are arranged in series, and the number of mixing devices 20 or the number of reaction devices 30 does not need to be plural. In addition, from the viewpoint of uniformly progressing the chemical reaction, the polymer production system 100 may include a microfluidic device different from the mixing device 20 or the reaction device 30. For example, a reaction device having an introduction unit capable of introducing the monomer BC solution into the reaction channel 6 may be provided.

Since the polymer production system 100 according to the present embodiment performs the mixing process and the chemical reaction process in separate devices, by performing temperature control for each device, it becomes possible to control the temperature of the fluid flowing through the microchannel separately for the mixing process and the chemical reaction process. In the present embodiment, by not heating the mixing device 20 and heating the reaction device 30 with the heater 40, the temperature of the fluid flowing through the microchannel can be changed for each device. In addition, since the microfluidic device has excellent temperature responsiveness, temperature control for each device is easy. Therefore, the temperature of the fluid flowing through the microchannel can be quickly switched for each device.

In addition, from the above viewpoint, the introduction unit 25 only needs to introduce at least one of the polymerization initiator A solution and the monomer BC solution, which are a plurality of different fluids, into the mixing channel 2b so that the reactants are contained in the mixed liquid, and, for example, only the monomer BC solution or only the polymerization initiator A solution may be introduced into the mixing channel 2b.

The polymer production system 100 according to the present embodiment includes a plurality of mixing devices 20 and reaction devices 30, and by introducing the monomer B and the monomer C into the production flow channel F1 multiple times and performing the polymerization reaction, the number of polymerization reaction processes performed in the production flow channel F1 can be increased, and the production amount of the polymer D, which is the reaction product, can be increased.

Furthermore, in the polymer production system 100 according to the present embodiment, the plurality of mixing devices 20 and the plurality of reaction devices 30 are arranged alternately in series. As a result, the mixed liquid supplied to each reaction device 30 is one uniformly mixed by the mixing device 20 adjacent upstream of the reaction device 30. Therefore, the reaction in each reaction device 30 can be progressed uniformly. Here, the "alternately arranged" means that the mixing devices 20 and the reaction devices 30 are arranged alternately in the sequence of the mixing devices 20 and the reaction devices 30, and does not intend to exclude the possibility that another type of device is interposed between the mixing device 20 and the reaction device 30. For example, a device that performs extraction or other chemical, biological, or physical processes as a unit operation on the fluid flowing through the microchannel may be interposed between the mixing device 20 and the reaction device 30. In addition, in the polymer production system 100, it is not essential that the plurality of mixing devices 20 and the plurality of reaction devices 30 are arranged alternately, and, for example, the reaction devices 30 may be arranged continuously in the flow direction. In such a case, an introduction unit capable of introducing the monomer BC solution into the reaction channel 6 may be provided in the reaction device 30 that is not adjacent upstream to the mixing device 20. In addition, the mixing devices 20 may be arranged continuously in the flow direction.

Here, in the polymer production system 100 including a plurality of mixing devices 20, since the polymerization initiator A solution and the monomer BC solution are continuously supplied (introduced) into the production flow channel F1 composed of the mixing channel 2b and the reaction channel 6 arranged in series, the flow rate of the fluid at any position in the production flow channel F1 in the flow direction becomes the sum of the flow rates of all the fluids introduced into the production flow channel F1 upstream of that position. For example, among the plurality of mixing devices 20, the mixing device 20 of the device unit 10 arranged at the q-th position (q is an integer of 2 or more and N or less) from the upstream side is referred to as the mixing device 20q. At this time, the total flow rate (total volume) of the fluid flowing through the mixing channel 2b of the mixing device 20q is approximately the sum of the flow rate (volume) of the fluid flowing through the mixing channel 2b of the mixing device 201 positioned at the most upstream and the flow rates (volumes) of the fluids additionally introduced in all the mixing devices 20 from the mixing device 201 to the mixing device 20q. Therefore, the total flow rate of the fluid flowing through the production flow channel F1 increases as the fluid passes through each mixing channel 2b from the upstream side to the downstream side. As a result, in the plurality of mixing devices 20, the total flow rate of the fluid flowing through the mixing channel 2b becomes larger in the mixing devices 20 located further downstream. Therefore, from the viewpoint of uniformly mixing the fluid in the channel and uniformly progressing the chemical reaction, it is preferable that the residence time in the mixing channel 2b of the mixing device 20 located further downstream be longer.

Furthermore, in the polymer production system 100 according to the present embodiment, in which the polymerization reaction of the monomer is performed, since multiple polymerization reaction processes are carried out in the production flow channel F1 by the additional charges of the monomer in the mixing device 20, the number of polymer molecules of the polymer D flowing through the production flow channel F1 increases each time the fluid passes through each reaction channel 6 from the upstream side to the downstream side. Since the concentration of the polymer D in the mixing channel 2b becomes higher in the mixing devices 20 located further downstream, depending on the polymer concentration and the like, the viscosity of the fluid flowing through the mixing channel 2b increases and the diffusion coefficient tends to decrease. That is, from the viewpoint of increasing the certainty of uniform mixing of the fluid in the channel, it is preferable that the residence time in the mixing channel 2b of the mixing device 20 located further downstream be longer. However, the diffusion coefficient is a factor that has a smaller influence on the mixing of the fluid than the total flow rate of the fluid.

In contrast, in the polymer production system 100 according to the present embodiment, the flow channel length L1 of the mixing channel 2b is set so that the flow channel length L1 becomes longer in the mixing devices 20 located further downstream. That is, in the plurality of mixing devices 20, the volume of each mixing channel 2b is set so that the volume of the mixing channel 2b becomes larger in the mixing devices 20 located further downstream. Since the residence time of the fluid in the channel is proportional to the volume of the channel, the residence time of the fluid in the mixing channel 2b becomes longer in the mixing devices 20 disposed further downstream. According to this, even in the mixing devices 20 arranged further downstream, the residence time necessary for uniformly mixing the fluid can be ensured. Therefore, even in the mixing devices 20 arranged further downstream, the fluid can be uniformly mixed, and the polymerization reaction in the reaction devices 30 arranged further downstream can proceed uniformly. As a result, according to the polymer production system 100 of the present embodiment, while introducing the monomer B and the monomer C by the plurality of mixing devices 20, the fluid can be uniformly mixed, and the quality of the polymer D can be ensured while increasing the production amount of the polymer D.

In the above viewpoint of uniformly mixing in the plurality of mixing devices 20, it is not essential that the volume becomes larger in all of the mixing devices 20 located further downstream, and it is sufficient that, in at least two of the plurality of mixing devices 20, the volume is set so that the volume of the mixing channel 2b becomes larger in the mixing devices 20 located further downstream. For example, among the plurality of mixing devices 20, the volume of any mixing device 20 may be equal to or smaller than the volume of any mixing device 20 disposed upstream of that mixing device 20. However, from the above viewpoint, it is more preferable that, in all of the mixing devices 20, the volume is set to become larger in the mixing devices 20 located further downstream.

In addition, for any mixing device 20 among the plurality of mixing devices 20, when the flow channel length [m] of the mixing channel 2b is L1, the total flow rate [m³/s] of the fluid flowing through the mixing channel 2b is u, and the diffusion coefficient [m²/s] of the fluid flowing through the mixing channel 2b is D, it is preferable from the above viewpoint that the flow channel length L1 of the mixing channel 2b be set so that L1 ≥ u/2D.

In the present embodiment, the mode is such that the flow rate in the mixing channel 2b located further downstream increases because the monomer solution is sequentially added by the plurality of mixing devices 20; however, the technology according to the present disclosure is not limited to such a mode, and the flow rate does not necessarily have to increase. For example, when a device that performs phase separation such as extraction as a unit operation is interposed between the mixing device and the reaction device, the flow rate decreases. In addition, the difficulty of uniform mixing in the mixing channel of the mixing device changes depending on various conditions such as not only the flow rate but also the chemical, biological, or physical processes performed in other devices and the characteristics or amounts of the reactants. From this, in the above viewpoint of uniformly mixing in the plurality of mixing devices, the technology according to the present disclosure does not make the volumes of the mixing channels in the plurality of mixing devices all the same, and may make the flow channel length of the mixing channel in at least one mixing device different from the volume of the mixing channel in another mixing device. According to this, the residence time in the mixing channel can be set according to the conditions of the mixing performed in the mixing channel. In addition, by adjusting the residence time for each mixing channel, the quality of the reaction product can be improved. The polymer production system 100 according to the present embodiment makes the volume of the mixing channel 2b in at least one mixing device 20 among the plurality of mixing devices different from the volume of the mixing channel 2b in another mixing device 20 in response to the increase in the flow rate in the mixing channel 2b located further downstream. In addition, in the technology according to the present disclosure, the volumes of the mixing channels of at least two mixing devices among the plurality of mixing devices may be the same. Furthermore, in the technology according to the present disclosure, the volume of the mixing channel may be set to become smaller in the mixing devices located further downstream depending on various conditions such as the types of chemical, biological, or physical processes and properties of the reactants.

In addition, in the present embodiment, the volumes of the mixing channels 2b are made different in two or more mixing devices 20 by making the flow channel lengths L1 different while making the cross-sectional areas of the mixing channels 2b the same; however, the technology according to the present disclosure is not limited thereto. In making the volumes of the mixing channels different in two or more mixing devices, it is not essential to make the flow channel lengths different. The technology according to the present disclosure may make the volumes of the mixing channels different by making the cross-sectional areas different while making the flow channel lengths of the mixing channels the same in two or more mixing devices, or may make the volumes of the mixing channels different by making both the cross-sectional areas and the flow channel lengths of the mixing channels different.

Furthermore, in the polymer production system 100 according to the present embodiment, by including the heater 40 as a reaction-promoting unit, the polymerization reaction of the monomer B and the monomer C in the reaction channel 6 can be promoted.

In addition, in the above embodiment, the polymerization initiator A solution and the monomer BC solution are mixed as the plurality of different fluids; however, the plurality of different fluids may include only a fluid containing the polymerization initiator and a fluid containing one kind of monomer. In the mixing channel 2b of the mixing device 20, a fluid containing one kind of monomer may be introduced from the introduction unit 25, and in the reaction channel 6 of the reaction device 30, the one kind of monomer may be polymerized in the presence of the polymerization initiator.

Although the above embodiment performs the polymerization reaction of the monomer B and the monomer C introduced into the mixing device, the technology according to the present disclosure is not limited to reactions between reactants contained in the different fluids (such as polymerization between monomers). The technology according to the present disclosure may, for example, mix a fluid containing a reactant and a fluid containing a decomposition catalyst as the plurality of different fluids in the mixing device, and perform a decomposition reaction of the reactant in the reaction device.

In the above embodiment, a chip-type microfluidic device is used as the microfluidic device; however, a tube-type microfluidic device or another form of microfluidic device may also be used, and, for example, the reaction device 30 may be configured as a tube-type microfluidic device.

### [Examples]

Hereinafter, the present disclosure is described in detail with reference to Examples. However, the present disclosure is not limited to aspects of Examples described below.

The methods for measuring the weight-average molecular weight Mw and the number-average molecular weight Mn of the polymers obtained in the following Examples and Comparative Examples are as follows. In addition, the polydispersity index Mw/Mn of the obtained polymers was calculated from the measured weight-average molecular weight Mw and number-average molecular weight Mn.

[Method for measuring weight-average molecular weight Mw and number-average molecular weight Mn]

The weight-average molecular weight Mw and the number-average molecular weight Mn of the polymers were measured by gel permeation chromatography (GPC). The measurement conditions for the GPC are as follows.
Apparatus: GPC system (manufactured by Shimadzu Corporation)
System controller: SIL-20A (manufactured by Shimadzu Corporation)
Pump: LC-20AD (manufactured by Shimadzu Corporation)
Degasser: DGU-20A3R (manufactured by Shimadzu Corporation)
Column oven: CTO-20AC (manufactured by Shimadzu Corporation)
RI detector: RID-20A (manufactured by Shimadzu Corporation)
Column: GPC KF-806L (column size: 8.0 mm (ID) × 300 mm (L), manufactured by Resonac Corporation) × 3 columns
Guard column: KF-G (column size: 4.6 mm (ID) × 10 mm (L), manufactured by Resonac Corporation)
Column temperature: 40°C
Cell temperature: 40°C
Eluent: tetrahydrofuran
Eluent flow rate: 0.8 mL/min
Injection volume: 35 µL
Analysis time: 60 min
Sample: 5 wt% tetrahydrofuran solution
Calibration standard: polystyrene calibration kit S-M-10 (manufactured by Agilent Technologies, Inc.)

### [Example 1]

A polymer was produced by a radical polymerization reaction of a monomer using a polymer production system 100 (N = 5) illustrated in Fig. 1. Table 1 shows the channel designs of mixing devices 201 to 205 and reaction devices 301 to 305 in the polymer production system 100. In addition, liquid-feeding pumps 60a and 60b of the polymer production system 100 are syringe pumps, and conduits 80a to 80e are polyether ether ketone tubes (inner diameter: 260 µm).

Specific operations, mixing conditions, and reaction conditions are as follows.

Raw material tanks 50a and 50b of the polymer production system 100 were filled with fluids described below, and each fluid was introduced into the respective mixing devices 201 to 205 and reaction devices 301 to 305 at flow rates shown in Table 2. The temperature during mixing in the mixing devices 201 to 205 was room temperature (25°C). In the reaction devices 301 to 305, a reaction channel 6 was heated by a heater 40 so that the reaction temperature became 95°C. Note that Table 2 also shows the diffusion coefficient D of methyl methacrylate (MMA, 25°C) in the mixed fluid adjusted in each of the mixing devices 201 to 205. The diffusion coefficient D was calculated in accordance with the Stokes-Einstein equation with reference to measurements by 1H-NMR and literature values.

### Liquid 1a supplied to the mixing device 201 (fluid stored in the raw material tank 50a)

A liquid (liquid 1a; solvent concentration: 80.0 mass%) prepared by dissolving dimethyl 2,2'-azobis(2-methylpropionate) (V-601; manufactured by FUJIFILM Wako Pure Chemical Corporation; polymerization initiator) in propylene glycol monomethyl ether acetate (MMPGAC; manufactured by FUJIFILM Wako Pure Chemical Corporation; boiling point: 145°C, melting point: below -10°C, specific gravity: 0.97; solvent) such that the concentration of the polymerization initiator became 20.0 mass%.

### Liquid 1b supplied to the mixing device 201 (fluid stored in the raw material tank 50b)

A liquid (liquid 1b; solvent concentration: 66.0 mass%) prepared by dissolving methyl methacrylate (MMA; manufactured by FUJIFILM Wako Pure Chemical Corporation; boiling point: 101°C, melting point: -48°C, specific gravity: 0.94) such that the concentration of methyl methacrylate became 17.0 mass%, and phenyl methacrylate (PhMA; manufactured by FUJIFILM Wako Pure Chemical Corporation; boiling point: 198°C, melting point: -17°C, specific gravity: 1.06) such that the concentration of phenyl methacrylate became 10.2 mass%, in propylene glycol monomethyl ether acetate (MMPGAC; same as described above).

### Liquid 1c supplied to the mixing devices 202 to 205 (fluid stored in the raw material tank 50b)

A liquid (liquid 1c; solvent concentration: 71.0 mass%) prepared by dissolving methyl methacrylate (MMA; manufactured by FUJIFILM Wako Pure Chemical Corporation; boiling point: 101°C, melting point: -48°C, specific gravity: 0.94) such that the concentration of methyl methacrylate became 12.0 mass%, and phenyl methacrylate (PhMA; manufactured by FUJIFILM Wako Pure Chemical Corporation; boiling point: 198°C, melting point: -17°C, specific gravity: 1.06) such that the concentration of phenyl methacrylate became 17.0 mass%, in propylene glycol monomethyl ether acetate (MMPGAC; same as described above).

The weight-average molecular weight Mw, the number-average molecular weight Mn, and the polydispersity index Mw/Mn of the polymer stored in the product tank 70 through the conduit 80e from the reaction device 305 were determined by the above method. The monomer conversion of the resulting polymer was also calculated. The results are shown in Table 7.

### [Example 2]

A polymer was produced in the same manner as in Example 1 except that the reaction temperature was changed to 90°C. The weight-average molecular weight Mw, the number-average molecular weight Mn, and the polydispersity index Mw/Mn of the resulting polymer were determined by the above method. The monomer conversion of the resulting polymer was also calculated. The results are shown in Table 7.

### [Comparative Example 1]

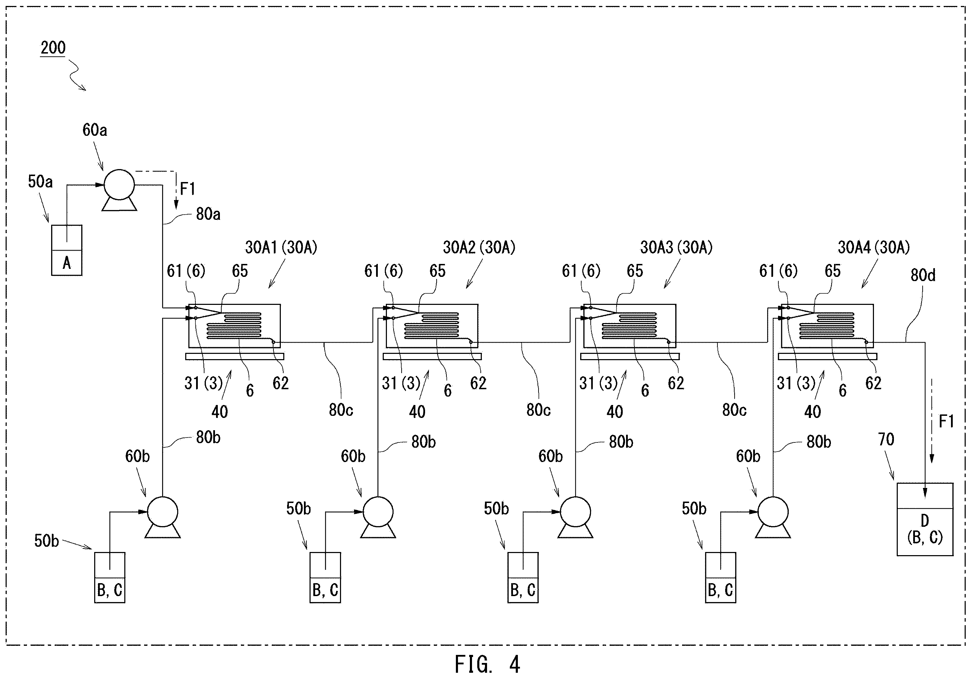

A polymer was produced by a radical polymerization reaction of a monomer using a polymer production system 200 illustrated in Fig. 4. The polymer production system 200 does not have the mixing devices used in the polymer production system of Example 1, and instead has four reaction devices 30A (30A1 to 30A4) arranged in series and connected by the conduit 80c. Each of the reaction devices 30A1 to 30A4 is provided with a supply port 61 for introducing a monomer solution into the reaction channel 6. Table 3 shows the channel designs of the reaction devices 30A1 to 30A4 in the polymer production system 200. In addition, liquid-feeding pumps 60a and 60b of the polymer production system 200 are syringe pumps, and the conduits 80a to 80d are polyether ether ketone tubes (inner diameter: 260 µm).

### Specific operations and reaction conditions are as follows.

Raw material tanks 50a and 50b of the polymer production system 200 were filled with an initiator solution and a monomer solution described below. From these raw material tanks, the initiator solution and the monomer solution were introduced into the respective reaction devices 30A1 to 30A4 at flow rates shown in Table 4, and a chemical reaction was performed. At that time, in the reaction devices 30A1 to 30A4, the reaction channel 6 was heated by the heater 40 such that the reaction temperature became 75°C.

### Liquid 2a supplied to the reaction device 30A1 (fluid stored in the raw material tank 50a)

A liquid (liquid 2a; solvent concentration: 58.0 mass%) prepared by dissolving dimethyl 2,2'-azobis(2-methylpropionate) (V-601; manufactured by FUJIFILM Wako Pure Chemical Corporation; polymerization initiator) in propylene glycol monomethyl ether acetate (MMPGAC; manufactured by FUJIFILM Wako Pure Chemical Corporation; boiling point: 145°C, melting point: below -10°C, specific gravity: 0.97; solvent) such that the concentration of the polymerization initiator became 42.0 mass%.

### Liquid 2b supplied to the reaction device 30A1 (fluid stored in the raw material tank 50b)

A liquid (liquid 2b; solvent concentration: 80.0 mass%) prepared by dissolving methyl methacrylate (MMA; manufactured by FUJIFILM Wako Pure Chemical Corporation; boiling point: 101°C, melting point: -48°C, specific gravity: 0.94) such that the concentration of methyl methacrylate became 9.8 mass%, and phenyl methacrylate (PhMA; manufactured by FUJIFILM Wako Pure Chemical Corporation; boiling point: 198°C, melting point: -17°C, specific gravity: 1.06) such that the concentration of phenyl methacrylate became 10.2 mass%, in propylene glycol monomethyl ether acetate (MMPGAC; same as described above).

### Liquid 2c supplied to the reaction devices 30A2 to 30A4 (fluid stored in the raw material tank 50b)

A liquid (liquid 2c; solvent concentration: 85.00 mass%) prepared by dissolving phenyl methacrylate (PhMA; manufactured by FUJIFILM Wako Pure Chemical Corporation; boiling point: 198°C, melting point: -17°C, specific gravity: 1.06) such that the concentration of phenyl methacrylate became 15.0 mass% in propylene glycol monomethyl ether acetate (MMPGAC; same as described above).

The weight-average molecular weight Mw, the number-average molecular weight Mn, and the polydispersity index Mw/Mn of the polymer stored in the product tank 70 through the conduit 80d from the reaction device 30A4 were determined by the above method. The monomer conversion of the resulting polymer was also calculated. The results are shown in Table 7.

### [Reference Example 1]

A polymer was produced by a radical polymerization reaction of a monomer using the same polymer production system as in Comparative Example 1, except that the number of reaction devices was one and the inner diameter of the polyether ether ketone tube serving as the conduit was 500 µm. Table 5 shows the channel design of the reaction device in the polymer production system.

Specific operations and reaction conditions are as follows.

The raw material tanks of the polymer production system were filled with an initiator solution and a monomer solution described below. From these raw material tanks, the initiator solution and the monomer solution were introduced into the reaction device at flow rates shown in Table 6, and a chemical reaction was performed. At that time, in the reaction device, the reaction channel was heated by the heater such that the reaction temperature became approximately 95 ± 1°C.

### Liquid 4a supplied to the reaction device (fluid stored in the raw material tank)

A liquid (liquid 4a; solvent concentration: 70.0 mass%) prepared by dissolving dimethyl 2,2'-azobis(2-methylpropionate) (V-601; manufactured by FUJIFILM Wako Pure Chemical Corporation; polymerization initiator) in propylene glycol monomethyl ether acetate (MMPGAC; manufactured by FUJIFILM Wako Pure Chemical Corporation; boiling point: 145°C, melting point: below -10°C, specific gravity: 0.97; solvent) such that the concentration of the polymerization initiator became 30.0 mass%.

### Liquid 4b supplied to the reaction device (fluid stored in the raw material tank)

A liquid (liquid 4b; solvent concentration: 71.4 mass%) prepared by dissolving methyl methacrylate (MMA; manufactured by FUJIFILM Wako Pure Chemical Corporation; boiling point: 101°C, melting point: -48°C, specific gravity: 0.94) such that the concentration of methyl methacrylate became 12.0 mass%, and phenyl methacrylate (PhMA; manufactured by FUJIFILM Wako Pure Chemical Corporation; boiling point: 198°C, melting point: -17°C, specific gravity: 1.06) such that the concentration of phenyl methacrylate became 16.6 mass%, in propylene glycol monomethyl ether acetate (MMPGAC; same as described above).

The weight-average molecular weight Mw, the number-average molecular weight Mn, and the polydispersity index Mw/Mn of the polymer stored in the product tank through the conduit from the reaction device were determined by the above method. The monomer conversion of the resulting polymer was also calculated. The results are shown in Table 7.

### [Table 1]

**Table 1**

| | Mixing device | | | | | Reaction device | | | | |
|---|---|---|---|---|---|---|---|---|---|---|
| | 201 | 202 | 203 | 204 | 205 | 301 | 302 | 303 | 304 | 305 |
| Channel depth (mm) | 0.5 | 0.5 | 0.5 | 0.5 | 0.5 | 0.5 | 0.5 | 0.5 | 0.5 | 0.5 |
| Channel width (mm) | 0.5 | 0.5 | 0.5 | 0.5 | 0.5 | 0.5 | 0.5 | 0.5 | 0.5 | 0.5 |
| Channel length L1 (mm) | 351 | 351 | 610 | 610 | 610 | 88 | 372 | 664 | 1,396 | 1,416 |
| Channel volume (µL) | 88 | 88 | 153 | 153 | 153 | 22 | 93 | 166 | 349 | 354 |

### [Table 2]

**Table 2**

| Device unit | Fluid | Supply port | Flow rate | |
|---|---|---|---|---|
| | | | (µL/min) | (m³/s) |
| 101 | Liquid 1a | 21 | 12.4 | 2.1×10⁻¹⁰ |
| | Liquid 1b | 31 | 14.4 | 2.4×10⁻¹⁰ |
| | Liquid 1c | 31 | 0.0 | 0.0 |
| | Mixed fluid prepared in mixing device 201 and discharged from discharge port 22 (diffusion coefficient D = 1.2 × 10⁻⁹[m²/s]) | 61 | 26.8 | 4.5×10⁻¹⁰ |
| 102 | Mixed fluid discharged from discharge port 62 of reaction device 301 | 21 | 26.8 | 4.5x10⁻¹⁰ |
| | Liquid 1b | 31 | 0.0 | 0.0 |
| | Liquid 1c | 31 | 18.6 | 3.1×10⁻¹⁰ |
| | Mixed fluid prepared in mixing device 202 and discharged from discharge port 22 (diffusion coefficient D = 1.2 × 10⁻⁹[m²/s]) | 61 | 45.4 | 7.6×10⁻¹⁰ |
| 103 | Mixed fluid discharged from discharge port 62 of reaction device 302 | 21 | 45.4 | 7.6×10⁻¹⁰ |
| | Liquid 1b | 31 | 0.0 | 0.0 |
| | Liquid 1c | 31 | 16.5 | 2.8×10⁻¹⁰ |
| | Mixed fluid prepared in mixing device 203 and discharged from discharge port 22 (diffusion coefficient D = 1.2 × 10⁻⁹[m²/s]) | 61 | 61.9 | 10.4×10⁻¹⁰ |
| 104 | Mixed fluid discharged from discharge port 62 of reaction device 303 | 21 | 61.9 | 10.4×10⁻¹⁰ |
| | Liquid 1b | 31 | 0.0 | 0.0 |
| | Liquid 1c | 31 | 8.2 | 1.4×10⁻¹⁰ |
| | Mixed fluid prepared in mixing device 204 and discharged from discharge port 22 (diffusion coefficient D = 1.2 × 10⁻⁹[m²/s]) | 61 | 70.1 | 11.8×10⁻¹⁰ |
| 105 | Mixed fluid discharged from discharge port 62 of reaction device 304 | 21 | 70.1 | 11.8×10⁻¹⁰ |
| | Liquid 1b | 31 | 0.0 | 0.0 |
| | Liquid 1c | 31 | 4.1 | 0.7×10⁻¹⁰ |
| | Mixed fluid prepared in mixing device 205 and discharged from discharge port 22 (diffusion coefficient D = 1.2 × 10⁻⁹[m²/s]) | 61 | 74.2 | 12.5×10⁻¹⁰ |

### [Table 3]

**Table 3**

| | Reaction device | | | |
|---|---|---|---|---|
| | 30A1 | 30A2 | 30A3 | 30A4 |
| Channel depth (mm) | 0.1 | 0.1 | 0.1 | 0.1 |
| Channel width (mm) | 0.25 | 0.25 | 0.25 | 0.25 |
| Channel length (mm) | 1,600 | 2,197 | 2,604 | 2,895 |
| Channel volume (µL) | 33.0 | 45.5 | 54.0 | 60.0 |

### [Table 4]

**Table 4**

| | Reaction device | | | |
|---|---|---|---|---|
| | 30A1 | 30A2 | 30A3 | 30A4 |
| Introduction amount of liquid 2a (µL/min) | 0.40 | - | - | - |
| Introduction amount of liquid 2b (µL/min) | 1.98 | - | - | - |
| Introduction amount of liquid 2c (µL/min) | - | 0.28 | 0.18 | 0.11 |
| Total flow rate (µL/min) | 2.38 | 2.66 | 2.84 | 2.95 |

### [Table 5]

**Table 5**

| | Reaction device |
|---|---|
| Channel depth (mm) | 0.5 |
| Channel width (mm) | 0.5 |
| Channel length (mm) | 1416 |
| Channel volume (µL) | 354 |

### [Table 6]

**Table 6**

| | Reaction device |
|---|---|
| Introduction amount of liquid 4a (µL/min) | 20.4 |
| Introduction amount of liquid 4b (µL/min) | 3.9 |
| Total flow rate (µL/min) | 24.3 |

### [Table 7]

**Table 7**

| | Weight-average molecular weight | Number-average molecular weight | Polydispersity index | Monomer conversion |
|---|---|---|---|---|
| | Mw | Mn | Mw/Mn | (%) |
| Example 1 | 4,160 | 2,631 | 1.58 | 82 |
| Example 2 | 5,666 | 3,348 | 1.69 | 77 |
| Comparative Example 1 | 7,281 | 3,683 | 1.98 | 90 |
| Reference Example 1 | 6,200 | 3,370 | 1.84 | 88 |

### [Modification Example]

A polymer production system 100A according to a modification example of the present embodiment is described below. In the following description of the modification example, differences from the polymer production system 100 described with reference to Figs. 1 to 3 are primarily described, and detailed descriptions of the same points as those of the polymer production system 100 are omitted.

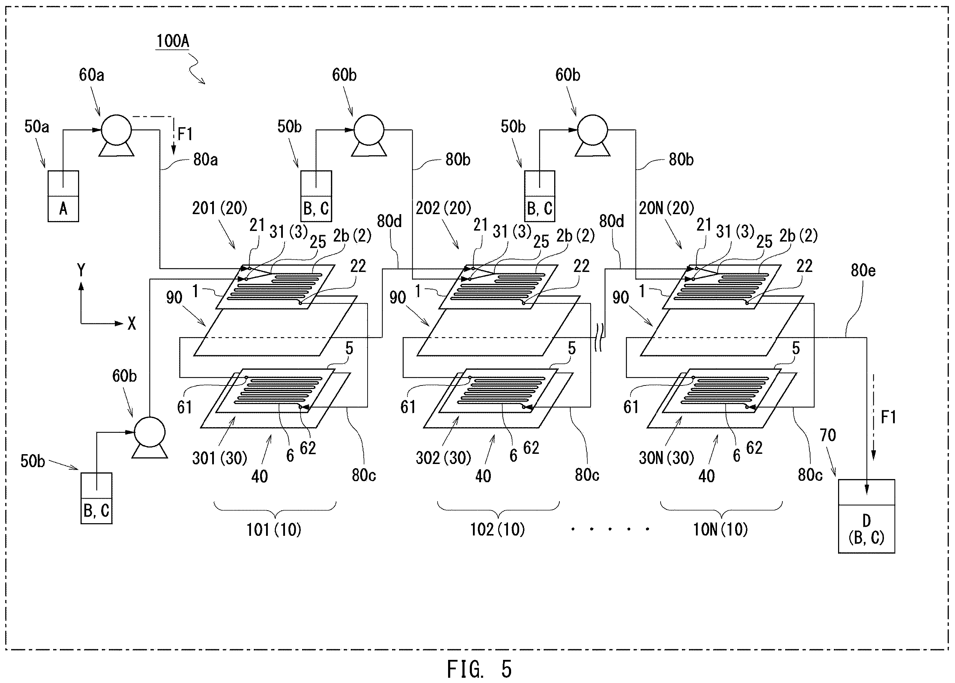

Fig. 5 is a configuration diagram of the polymer production system 100A according to the modification example of the present embodiment. As illustrated in Fig. 5, the polymer production system 100A according to the modification example has a two-story hierarchical structure and differs from the polymer production system 100 described above in that a mixing device 20 and a reaction device 30 are arranged side by side in the up-and-down direction (vertical direction) in a device unit 10. Furthermore, the polymer production system 100A according to the modification example differs from the polymer production system 100 described above by including a partition member 90 disposed between the mixing device 20 and the reaction device 30.

Here, as illustrated in Fig. 5, a direction parallel to a plane in which the microfluidic device extends (i.e., the upper and lower surfaces of the substrate of the microfluidic device) is defined as an X direction, and a direction perpendicular to the plane in which the microfluidic device extends is defined as a Y direction. The Y direction is a direction orthogonal to the substrate of the microfluidic device. The X direction and the Y direction are orthogonal to each other. Each microfluidic device of the polymer production system 100A according to the modification example is arranged such that the X direction coincides with the horizontal direction and the Y direction coincides with the vertical direction. However, the present disclosure is not limited thereto.

As illustrated in Fig. 5, in the modification example, a plurality of mixing devices 20 and a plurality of reaction devices 30 are arranged alternately in series such that the mixing device 20 and the reaction device 30 that are adjacent to each other in the flow direction can communicate with each other, similarly to the polymer production system 100 of Fig. 1. A heater 40 is provided together with the reaction device 30. In the modification example, in a device unit 10, which is a pair of the mixing device 20 and the reaction device 30 that are adjacent to each other in the flow direction, the mixing device 20 and the reaction device 30 are arranged side by side in the Y direction. Specifically, in the device unit 10, the mixing device 20 and the reaction device 30 are arranged at different positions in the Y direction and at the same position in the X direction. In other words, in the device unit 10, the mixing device 20 and the reaction device 30 are arranged facing each other with a spacing in the Y direction. In the modification example, a plurality of device units 10 are arranged side by side in the X direction. Accordingly, in the polymer production system 100A according to the modification example, the mixing device 20 and the reaction device 30 that are adjacent to each other in the flow direction are arranged at different positions in the Y direction.

In the polymer production system 100A according to the modification example, the microfluidic devices may be arranged, for example, the microfluidic devices may be arranged, for example, in a two-tier rack with upper and lower tiers. In the modification example, the mixing device 20 is arranged on the upper tier, and the reaction device 30 is arranged on the lower tier. However, the present disclosure is not limited thereto, and the mixing device 20 may be arranged on the lower tier while the reaction device 30 on the upper tier. In addition, conduits 80c and 80d that connect the microfluidic devices to each other may be connected, for example, perpendicular to a side surface of the substrate of each microfluidic device, thereby allowing the lengths of conduits 80c and 80d to be reduced compared with a case where the upper surfaces or lower surfaces of the substrates of the microfluidic devices are connected to each other by conduits 80c and 80d.

Furthermore, as illustrated in Fig. 5, a partition member 90 having heat-insulating properties is disposed between the mixing device 20 and the reaction device 30 in the device unit 10. The partition member 90 is formed in a plate shape and is arranged in a direction orthogonal to the Y direction. That is, the partition member 90 is arranged in a direction parallel to the microfluidic device. The partition member 90 is formed of a material having heat-insulating properties. Although the material of the partition member 90 is not particularly limited, a material having excellent heat-insulating properties, such as a resin material, may be used.

As described above, in the polymer production system 100A according to the modification example, the mixing device 20 and the reaction device 30 that are adjacent to each other in the flow direction are arranged side by side in the Y direction. Accordingly, heat generated by the heater 40 provided together with the reaction device 30 is less likely to be transferred to the mixing device 20. Therefore, the influence of heating by the heater 40 on the mixing device 20 is reduced, and mixing in the mixing device 20 can be made more uniform. Moreover, the polymer production system 100A according to the modification example includes the partition member 90 having heat-insulating properties that is disposed between the mixing device 20 and the reaction device 30, thus enabling a further reduction in the influence of heat on the mixing device 20.

The aspect according to the modification example is applicable not only to the reaction device 30 but also to a case where an other-process device different from the reaction device 30 is used. Even in such a case, for example, the influence of a reaction-promoting unit, such as a heater provided together with the other-process device, on the mixing device 20 is suppressed, and mixing in the mixing device 20 can be made more uniform. In addition, the present disclosure does not require that all of the mixing devices and the other-process devices that are adjacent to each other in the flow direction be arranged side by side in the Y direction. In at least one pair of a mixing device and an other-process device that are adjacent to each other in the flow direction, the mixing device and the other-process device may be arranged side by side in the direction perpendicular to the plane in which the microfluidic device extends.

### Description of the Reference Numerals and Symbols

2b mixing channel
10 mixing device
40 heater (example of reaction-promoting unit)
100 polymer production system

## Claims

1. A microfluidic system comprising a plurality of chip-type microfluidic devices in which a microchannel is formed, the microfluidic system comprising:
one or more mixing devices, each of which is a microfluidic device that comprises a mixing channel configured to mix a plurality of different fluids and an introduction unit configured to introduce at least one of the plurality of different fluids into the mixing channel; and
one or more other-process devices, each of which is a microfluidic device configured to perform, within a microchannel, a chemical, biological, or physical process other than a mixing process on a mixed fluid resulting from mixing the plurality of different fluids in the mixing channel.

2. The microfluidic system according to claim 1,
wherein the chemical, biological, or physical process performed in the other-process devices is at least one selected from chemical reaction, extraction, distillation, concentration, solid-phase extraction, particle separation or classification, crystallization, and cell culture.

3. The microfluidic system according to claim 1,
wherein the other-process devices are each a reaction device in which a chemical reaction is performed, the reaction device comprising a reaction channel configured to allow a chemical reaction of a reactant contained in the mixed fluid resulting from mixing the plurality of different fluids in the mixing channel to proceed; and
wherein the one or more mixing devices and the one or more reaction devices are arranged in series such that the mixing channel and the reaction channel communicate with each other.

4. The microfluidic system according to claim 3, comprising a plurality of the mixing devices and a plurality of the reaction devices,
wherein the plurality of mixing devices and the plurality of reaction devices are arranged alternately in series.

5. The microfluidic system according to claim 3 or 4,
wherein the plurality of different fluids comprise at least a fluid containing a polymerization initiator and a fluid containing one or more monomers;
wherein the fluid containing the one or more monomers is introduced into the mixing channel of the mixing device from the introduction unit; and
wherein, in the reaction channel of the reaction device, the one or more monomers are polymerized in the presence of the polymerization initiator.

6. The microfluidic system according to claim 3 or 4, further comprising a reaction-promoting unit configured to promote a chemical reaction of a reactant contained in the mixed fluid in the reaction channel.

7. The microfluidic system according to claim 6, wherein the reaction-promoting unit comprises at least one selected from a heating unit, a light-irradiation unit, a vibration-energy-imparting unit, and a voltage-application unit.

8. The microfluidic system according to claim 3 or 4, wherein a channel width of the mixing channel and a channel width of the reaction channel are each 1000 µm or less.

9. The microfluidic system according to any one of claims 1 to 4,
wherein the one or more mixing devices and the one or more other-process devices are arranged in series; and
wherein, in at least one pair of a mixing device and an other-process device that are adjacent to each other in a flow direction, the mixing device and the other-process device are arranged side by side in a direction perpendicular to a plane in which the microfluidic device extends.

10. The microfluidic system according to claim 9, further comprising a partition member having heat-insulating properties, the partition member being disposed between the mixing device and the other-process device in the at least one pair of the mixing device and the other-process device.

11. A processing method comprising using a microfluidic system comprising a plurality of chip-type microfluidic devices in which a microchannel is formed,
the microfluidic system comprising:
one or more mixing devices, each of which is a microfluidic device that comprises a mixing channel configured to mix a plurality of different fluids and an introduction unit configured to introduce at least one of the plurality of different fluids into the mixing channel; and
one or more other-process devices, each of which is a microfluidic device configured to perform, within a microchannel, a chemical, biological, or physical process other than a mixing process on a mixed fluid resulting from mixing the plurality of different fluids in the mixing channel.

12. The processing method according to claim 11, wherein the chemical, biological, or physical process performed in the other-process devices is at least one selected from chemical reaction, extraction, distillation, concentration, solid-phase extraction, particle separation or classification, crystallization, and cell culture.

13. The processing method according to claim 11,
wherein the other-process devices are each a reaction device in which a chemical reaction is performed, the reaction device comprising a reaction channel configured to allow a chemical reaction of a reactant contained in the mixed fluid resulting from mixing the plurality of different fluids in the mixing channel to proceed; and
wherein the one or more mixing devices and the one or more reaction devices are arranged in series such that the mixing channel and the reaction channel communicate with each other.

14. The processing method according to claim 13,
wherein the microfluidic system comprises a plurality of the mixing devices and a plurality of the reaction devices; and
wherein the plurality of mixing devices and the plurality of reaction devices are arranged alternately in series.

15. The processing method according to claim 13 or 14,
wherein the plurality of different fluids comprise at least a fluid containing a polymerization initiator and a fluid containing one or more monomers;
wherein the fluid containing the one or more monomers is introduced into the mixing channel of the mixing device from the introduction unit; and
wherein, in the reaction channel of the reaction device, the one or more monomers are polymerized in the presence of the polymerization initiator.

16. The processing method according to claim 13 or 14, wherein the microfluidic system further comprises a reaction-promoting unit configured to promote a chemical reaction of a reactant contained in the mixed fluid in the reaction channel.

17. The processing method according to claim 16, wherein the reaction-promoting unit comprises at least one selected from a heating unit, a light-irradiation unit, a vibration-energy-imparting unit, and a voltage-application unit.

18. The processing method according to any one of claims 11 to 14,
wherein the one or more mixing devices and the one or more other-process devices are arranged in series; and
wherein, in at least one pair of a mixing device and an other-process device that are adjacent to each other in a flow direction, the mixing device and the other-process device are arranged side by side in a direction perpendicular to a plane in which the microfluidic device extends.

19. The processing method according to claim 18, wherein a partition member having heat-insulating properties is disposed between the mixing device and the other-process device in the at least one pair of the mixing device and the other-process device.

20. A method for producing a polymer, the method comprising using a microfluidic system comprising a plurality of chip-type microfluidic devices in which a microchannel is formed,
the microfluidic system comprising:
a plurality of mixing devices, each of which is a microfluidic device that comprises a mixing channel configured to mix a plurality of different fluids comprising at least a fluid containing a polymerization initiator and a fluid containing one or more monomers, and an introduction unit configured to introduce at least the one or more monomers among the plurality of different fluids into the mixing channel; and
one or more reaction devices, each of which is a microfluidic device that comprises a reaction channel configured to allow a polymerization reaction of the one or more monomers to proceed in the presence of the polymerization initiator by the plurality of different fluids mixed in the mixing channel,
wherein the plurality of mixing devices and the one or more reaction devices are arranged in series such that the mixing channel and the reaction channel communicate with each other.
